(19) Europäisches Patentamt
European Patent Office
Office européen des brevets

(11) EP 1 400 234 A1

(12) DEMANDE DE BREVET EUROPEEN

(43) Date de publication:
**24.03.2004 Bulletin 2004/13**

(21) Numéro de dépôt: **03292137.1**

(22) Date de dépôt: **29.08.2003**

(51) Int Cl.[7]: **A61K 7/02**, A61K 7/021, A61K 7/025, A61K 7/031, A61K 7/032, A61K 7/043, A61K 7/48, A61K 7/06

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **20.09.2002 FR 0211646**
**20.09.2002 FR 0211647**

(71) Demandeur: **L'OREAL**
**75008 Paris (FR)**

(72) Inventeur: **Jager Lezer, Nathalie**
**91000 Verrieres-le-Buisson (FR)**

(74) Mandataire: **Kromer, Christophe**
**L'OREAL - D.I.P.I.**
**25-29 Quai Aulagnier**
**92600 Asnières (FR)**

(54) **Composition cosmétique comprenant des fibres rigides et un composé choisi parmi un polymère filmogène et/ou une cire**

(57) L'invention a pour objet une composition cosmétique comprenant des fibres rigides sensiblement rectilignes et un composé choisi parmi un polymère filmogène et/ou une cire, la composition présentant un comportement plastique thixotrope.

La composition présente une bonne dispersion des fibres rigides et permet un dépôt homogène sur les matières kératiniques. En particulier, la composition confère un bon effet d'allongement des cils.

Application au maquillage et au soin des matières kératiniques, en particulier en mascara.

EP 1 400 234 A1

## Description

La présente invention a pour objet une composition comprenant, dans un milieu physiologiquement acceptable, des fibres rigides et un composé choisi parmi un polymère filmogène et/ou une cire, destinée en particulier au domaine cosmétique. L'invention a aussi pour objet un procédé de maquillage ou de soin cosmétique des matières kératiniques utilisant cette composition. La composition et le procédé selon l'invention sont plus particulièrement destinés aux matières kératiniques telles que la peau, y compris les lèvres, et les phanères comme les cils, les sourcils, les cheveux et les ongles, notamment d'êtres humains. Plus spécialement, l'invention porte sur un mascara.

La composition selon l'invention peut se présenter sous la forme d'un produit de revêtement des fibres kératiniques telles que les sourcils, les cheveux, les cils, et notamment sous la forme de composition de revêtement des cils (mascara), d'eye-liner, de produit pour les lèvres, de fard à joues ou à paupières, de fond de teint, de produit de maquillage du corps, de produit anti-cernes, de vernis à ongles, de produit de soin de la peau.

La composition de revêtement des fibres kératiniques peut être une composition de maquillage, une composition à appliquer sur un maquillage, dite encore top-coat, ou bien encore une composition de traitement (ou de soin) des cils, des sourcils ou des cheveux.

Il est connu, dans ce domaine de la technique, d'employer des fibres dans les compositions de maquillage ou de soin des matières kératiniques pour améliorer leurs propriétés cosmétiques.

Ainsi, il est connu du document JP-A-3 151 613 d'utiliser les fibres dans des compositions de mascara pour conférer un effet d'allongement et d'épaississement aux cils. Les documents JP-A-57 158 714 et JP-A-9 263 518 décrivent des compositions de mascara comprenant des fibres et des polymères de type acrylique en dispersion aqueuse.

De même, les documents JP-A-6 9340 et JP-A-7 179 323 décrivent des compositions de mascara comprenant des fibres, notamment des fibres de nylon, et des polymères filmogènes en dispersion aqueuse.

Le document FR-A-2 817 477 décrit des compositions ou formulations cosmétiques comprenant des fibres à base d'un polymère synthétique ou artificiel, tel que le polypropylène, le PET, le polyamide 6 et le polyamide 66.

Les compositions comprenant des fibres comprennent généralement un agent épaississant pour conférer à la composition une consistance permettant une application aisée de la composition sur les matières kératiniques. Or on a constaté que certains épaississants comme l'hydroxyéthylcellulose ne permettent pas d'obtenir une bonne dispersion des fibres dans la composition : les fibres sont alors mal réparties lors de l'application de la composition sur les matières kératiniques, les fibres sont donc orientées et réparties de manière aléatoire sur les matières kératiniques, et il en résulte un dépôt hétérogène nuisant aux bonnes propriétés cosmétiques attendues. En particulier, pour un mascara, la mauvaise dispersion des fibres conduit à effet d'allongement médiocre et non homogène.

En particulier, les compositions de mascara contenant des fibres ne permettent pas d'obtenir un effet d'allongement optimal et de longue durée car cet effet est perdu rapidement après l'application de la composition. En effet, les fibres ne se situent pas dans le prolongement des cils.

L'effet obtenu par les compositions de mascara contenant des fibres de l'art antérieur est souvent esthétiquement inacceptable, notamment dans le cas de cils fournis et/ou longs et/ou recourbés, pour lesquels on obtient un aspect des cils, dit « sapin de noël », particulièrement disgracieux.

Il existe donc un besoin pour une composition cosmétique, en particulier une composition de mascara qui permette d'obtenir d'excellentes propriétés cosmétiques, un maquillage homogène et précis.

Il existe notamment un besoin pour une composition de mascara qui procure un allongement parfait, dans une continuité parfaite du cil, un positionnement régulier, non-aléatoire, des fibres exactement dans le prolongement du cil et donc un effet esthétique optimal.

La composition doit, de plus, bien adhérer aux fibres kératiniques, par exemple aux cils, ne pas former de paquets et être d'une application facile et rapide.

Le but de la présente invention est, entre autres, de répondre aux besoins et de satisfaire aux exigences mentionnés plus haut.

Les inventeurs ont découvert qu'en utilisant des fibres particulières dans une composition comprenant au moins un composé choisi parmi un polymère filmogène et/ou une cire et présentant un comportement plastique thixotrope, on obtient une composition comprenant une dispersion homogène des fibres et conduisant donc à un maquillage homogène et précis des matières kératiniques, et notamment des cils. En particulier, lorsque la composition est un mascara, les fibres appliquées sur les cils s'orientent et se fixent dans le prolongement des cils , quelle que soit la typologie des cils. Les fibres ne sont donc pas disposées de manière aléatoire, mais se placent dans le prolongement des cils.

De façon plus précise, l'invention a pour objet une composition comprenant, dans un milieu physiologiquement acceptable, des fibres rigides sensiblement rectilignes et au moins un composé choisi parmi un polymère filmogène et/ou une cire, la composition ayant un comportement plastique thixotrope.

L'invention a également pour objet un procédé cosmétique de maquillage et de soin des matières kératiniques,

notamment des cils, comprenant l'application sur les matières kératiniques (notamment sur les cils) d'une composition telle que définie précédemment.

L'invention a également pour objet l'utilisation d'une composition telle que définie précédemment pour obtenir un dépôt, notamment un maquillage, homogène sur les matières kératiniques.

L'invention a encore pour objet l'utilisation de fibres rigides, sensiblement rectilignes, dans une composition comprenant, dans un milieu physiologiquement acceptable, au moins un polymère filmogène et/ou au moins une cire , la composition ayant un comportement plastique thixotrope, pour obtenir un dépôt, notamment un maquillage, homogène sur les matières kératiniques.

L'invention a également pour objet l'utilisation d'un mascara comprenant une composition telle que définie précédemment, pour obtenir un allongement dans le prolongement des cils et/ou pour mimer le prolongement des cils.

L'invention a aussi pour objet l'utilisation de fibres rigides, sensiblement rectilignes, dans une composition comprenant, dans un milieu physiologiquement acceptable, au moins un polymère filmogène et/ou au moins une cire, la composition ayant un comportement plastique thixotrope, pour obtenir un allongement dans le prolongement des cils et/ou pour mimer le prolongement des cils.

Conformément à l'invention, la composition comprend des fibres rigides, sensiblement rectilignes.

Sauf indication contraire, les valeurs des paramètres indiqués dans la présente demande sont mesurées à température ambiante (25°C).

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2 500, de préférence de 5 à 500, et mieux de 5 à 150.

Avantageusement, au moins 50 % en nombre, de préférence au moins 75 % en nombre, et mieux au moins 90 % en nombre des fibres sont telles que l'angle formé entre la tangente à l'axe central longitudinal de la fibre à l'une des extrémités de la fibre et la droite reliant ladite extrémité au point sur l'axe central longitudinal de la fibre correspondant à la moitié de la longueur de la fibre soit inférieur à 15 °,et l'angle formé entre la tangente à l'axe central longitudinal de la fibre en un point situé à mi-longueur de la fibre et la droite reliant l'une des extrémités au point sur l'axe central longitudinal de la fibre correspondant à la moitié de la longueur de fibre soit inférieur ou égal à 15 °, pour une même longueur de fibre allant de 0,8 mm à 5 mm, de préférence allant de 1 mm à 4 mm, de préférence allant de 1 mm à 3 mm, et mieux de 2 mm.

Avantageusement, l'angle, mentionné ci-dessus, est mesuré aux deux extrémités de la fibre et en un point situé à mi-longueur de la fibre, en d'autres termes, on effectue dans ce cas trois mesures et la moyenne des angles mesurés est inférieure ou égale à 15°.

Notamment, la tangente, en tout point de la fibre, forme un angle inférieur à 15°.

Dans la présente demande, l'angle formé par la tangente en un point de la fibre est l'angle formé entre la tangente à l'axe central longitudinal de la fibre audit point de la fibre et la droite reliant l'extrémité de la fibre la plus proche dudit point au point sur l'axe central longitudinal de la fibre correspondant à la moitié de la longueur de la fibre.

Généralement, les fibres incluses dans la composition ont la même longueur de fibre ou une longueur sensiblement identique.

Plus précisément, selon l'invention, lorsque l'on observe au microscope, avec un objectif permettant un grossissement de 2,5 et avec une vision plein champ, un milieu dans lequel sont dispersées les fibres à une concentration des fibres de 1 % en poids, un nombre majoritaire de fibres, c'est-à-dire au moins 50 % en nombre des fibres, de préférence au moins 75 % en nombre des fibres, et mieux au moins 90 % en nombre des fibres, doivent satisfaire à la condition angulaire définie plus haut. La mesure conduisant à la valeur de l'angle est effectuée pour une même longueur de fibres, cette longueur est comprise dans la gamme allant de 0,8 mm à 5 mm, de préférence de 1 à 4 mm, de préférence de 1 à 3 mm, et mieux de 2 mm.

Le milieu dans lequel est réalisée l'observation est un milieu dispersant assurant une bonne dispersion des fibres, par exemple de l'eau, un gel aqueux d'argile ou de polyuréthane associatif tels que décrits ultérieurement. On peut même réaliser une observation directe de la composition contenant les fibres. Un échantillon de la composition ou de la dispersion préparée est placée entre lame et lamelle pour l'observation au microscope avec un objectif permettant un grossissement de 2,5 et avec une vision plein champ. La vision plein champ permet de voir les fibres dans leur intégralité.

En fait, les fibres incluses dans les compositions de l'invention peuvent aussi être définies comme étant des fibres rigides, par opposition aux fibres des compositions de l'art antérieur, qui ne sont pas des fibres rigides et forment, de ce fait, des boucles de courbures assez importantes à l'observation au microscope.

En d'autres termes, les fibres des compositions de l'invention, initialement sensiblement droites, lorsqu'elles sont placées dans un milieu dispersant, ne voient pas leur forme sensiblement modifiée, ce qui se traduit par la condition angulaire définie ci-dessus, reflétant une forme que l'on peut qualifier de toujours, sensiblement droite, linéaire. Cette condition d'angle reflète la rigidité des fibres qui peut difficilement être exprimée par un autre paramètre que celui choisi selon l'invention pour des objets ayant une taille aussi faible que les fibres mise en oeuvre dans les compositions

de l'invention.
Plus exactement, la condition d'angle à laquelle doivent satisfaire les fibres de la composition de l'invention illustre la conservation de la forme des fibres, qui demeure sensiblement rectiligne, en raison de la rigidité de la fibre.
Avec les fibres de l'art antérieur, qui ne se présentent pas comme des « bâtonnets droits » et qui ne satisfont donc pas à la condition d'angle des fibres des compositions de l'invention, l'effet d'allongement des cils ne peut être obtenu et n'est donc pas optimisé.
Les fibres de l'art antérieur qui sont souples n'ont pas de forme initialement rectiligne et lorsqu'elles sont placées dans un milieu dispersant tel qu'une composition cosmétique, ces fibres se déforment en formant des boucles, et ne procurent aucun effet d'allongement, et donnent un aspect inesthétique du cil. Pour employer une comparaison triviale, les fibres selon l'invention peuvent être comparées à des spaghettis avant cuisson qui sont rigides, rectilignes et conservant cette forme alors que les fibres de l'art antérieur, souples, pourraient être comparées à des spaghettis cuits qui se déforment, se recourbent, et ne peuvent maintenir une forme rectiligne.

Les compositions de l'invention, incorporant les fibres spécifiques décrites plus haut, possèdent d'excellentes propriétés cosmétiques, en particulier elles permettent un maquillage homogène et précis, dans le cas des mascaras. En particulier, lorsque la composition est appliquée sur les cils, les fibres rigides ne sont pas disposées de manière aléatoire, mais se placent dans le prolongement des cils. En fait, la composition selon l'invention confère un très bon effet d'allongement aux cils, grâce aux fibres particulières qu'elle contient et qui se placent parfaitement, exactement, dans le prolongement du cil. La composition de l'invention permet, en fait, de réaliser une véritable « prothèse » du cil, ce qui est totalement impossible avec les compositions de l'art antérieur, renfermant des fibres non-rigides.

Les compositions de mascara, selon l'invention, assurent un parfait allongement et un effet esthétique optimal, même dans le cas des cils fournis et/ou longs et/ou recourbés, pour lesquels des effets particulièrement inesthétiques étaient obtenus avec les compositions de l'art antérieur, comprenant des fibres, se recourbant, non rigides.
Les compositions de mascara, selon l'invention, réalisent une continuité parfaite du cil, si bien que les fibres ne peuvent plus être observées à l'oeil nu car elles sont dans le prolongement exact du cil et « miment » parfaitement celui-ci.

Ces fibres peuvent être unitaires ou organisées par exemple tressées. Leur forme ou morphologie peut être quelconque et notamment de section circulaire ou polygonale (carrée, hexagonale ou octogonale) selon l'application spécifique envisagée. Avantageusement, la section transversale de la fibre (section perpendiculaire à l'axe de la direction de la longueur de la fibre) ne présente pas une plus grande longueur L1 et une plus petite longueur L2 (L2 correspond à l'épaisseur de la fibre) telle que L1/L2 (le rapport L1/L2 est encore appelé facteur d'aplatissement) est supérieur ou égal à 4.

En particulier, leurs extrémités sont épointées et/ou polies pour éviter de se blesser.

En particulier, les fibres peuvent avoir une longueur (L) allant de 0,8 mm à 5 mm, de préférence allant de 1 mm à 4 mm et mieux allant de 1 mm à 3 mm. Leur section peut être comprise dans un cercle de diamètre (D) allant de 2 nm à 500 µm, de préférence allant de 100 nm à 100 µm et mieux de 1 µm à 50 µm.

Le titre des fibres est souvent donné en denier. Le denier est le poids en gramme pour 9 km de fil. De préférence, les fibres utilisées selon l'invention ont un titre allant de 0,15 à 30 deniers, et mieux de 0,18 à 18 deniers.

Avantageusement, les fibres sont insolubles dans l'eau à 25 °C.

Les fibres rigides peuvent être choisies parmi les fibres d'un polymère synthétique choisi parmi les polyesters, les polyuréthanes, les polymères acryliques, les polyoléfines, les polyamides, en particulier les polyamides non aromatiques et les polyimides-amides aromatiques. Avantageusement, les fibres rigides ne sont pas des fibres comprenant plusieurs couches alternées de polymères ayant des indices de réfraction différents.

Comme exemples de fibres rigides, on peut citer les fibres :

- de polyesters, telles que celles obtenues par découpe de fils vendus sous les dénominations FIBRE 255-100-R11-242T TAILLE 3 MM (section octalobée), FIBRE 265-34-R11-56T TAILLE 3 MM (section ronde), FIBRE COOLMAX 50-34-591 TAILLE 3 MM (section tétralobée) par la société DUPONT DE NEMOURS ;

- de polyamide, telles que celles vendues sous les dénominations TRILOBAL NYLON 0.120-1.8 DPF ; TRILOBAL NYLON 0.120-18 DPF ; NYLON 0.120-6 DPF par la société Cellusuede products ; ou obtenues par découpe de fils vendus sous la dénomination FIBRE NOMEX BRAND 430 TAILLE 3 MM par la société . DUPONT DE NEMOURS ;

- de polyimide-amide telles que celles vendues sous les dénomination "KERMEL", "KERMEL TECH" par la société RHODIA ;

- de poly-(p-phénylène-téréphtalamide) (ou d'aramide) notamment vendues sous la dénomination Kevlar ® par la société DUPONT DE NEMOURS ;

- des fibres à structure multicouche comprenant des couches alternées de polymères choisis parmi les polyesters, les polymères acryliques, les polyamides, tellles que celles décrites dans les documents EP-A-6921217, EP-A-686858 et US-A-5472798. De telles fibres sont vendues sous les dénominations "Morphotex", « Teijin Tetron Morphotex » par la société TEIJIN.

Les fibres rigides particulièrement préfères sont les fibres de polyimide-amide aromatiques.

Les polyimides-amides aromatiques entrant dans la composition des fibres selon l'invention peuvent être tout polyimide-amide aromatique, mais ils comprennent généralement un motif récurrent répondant à la formule générale (1) suivante :

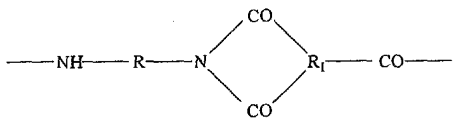

(I)

Ces polyimides-amides aromatiques comprennent, en outre, éventuellement, un motif récurrent (motif « amide ») de formule (II) :

$$-NH-R-NH-CO-R_2-CO- \quad (II)$$

Ces polyimides-amides aromatiques comprennent, en outre, éventuellement, un motif récurrent (motif « amide ») de formule (III) :

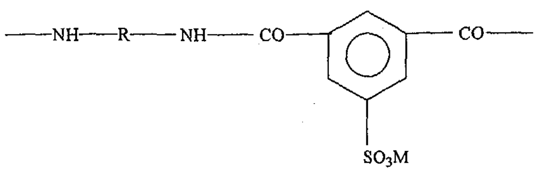

(III)

Ces polyimides-amides aromatiques comprennent, en outre, éventuellement, un motif récurrent (motif « amide ») de formule (IV) :

(IV)

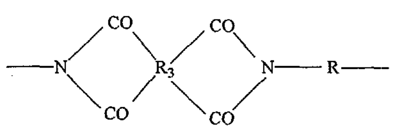

dans lesquelles R représente un groupe aromatique divalent, $R_2$ représente un groupe aromatique divalent, $R_3$ représente un groupe aromatique tétravalent, $R_1$ représente un groupe aromatique trivalent, et M représente un métal alcalin ou alcalino-terreux.

Par exemple, R et $R_2$ représentent chacun indépendamment un groupe divalent comprenant au moins un cycle aromatique, éventuellement substitué, ayant de 6 à 10 atomes de carbone et/ou un hétérocycle à caractère aromatique, éventuellement substitué, ayant de 5 à 10 atomes et comprenant un ou plusieurs hétéroatomes choisis parmi S, N et 0 ; $R_1$ représente un groupe trivalent comprenant au moins un cycle aromatique carboné, éventuellement substitué, ayant de 6 à 10 atomes de carbone et/ou un hétérocycle à caractère aromatique, éventuellement substitué, ayant de 5 à 10 atomes et comprenant un ou plusieurs hétéroatomes choisis parmi S, N et 0.

Dans la formule (1) citée ci-dessus, $R_1$ peut être, par exemple, un cycle benzénique éventuellement substitué, par un ou deux substituant(s) choisi(s) parmi les groupes alkyle et alcoxy de 1 à 10 C, les atomes d'halogène, le groupe nitro et le groupe sulfonyle ; ou plusieurs cycles benzéniques éventuellement substitués par un ou plusieurs substituant(s) choisi(s) parmi les groupes alkyle et alcoxy de 1 à 10 C, les atomes d'halogène, le groupe nitro et le groupe sulfonyle ; par exemple $R_1$ pourra comprendre de 2 à 5 cycles, reliés entre eux par une simple liaison ou par un groupe divalent, l'enchaînement desdits cycles pouvant être indépendamment en méta ou en para.

**[0042]** Ledit groupe divalent reliant lesdits cycles est choisi par exemple parmi :

- un groupe divalent dérivé d'un groupe alkyle linéaire ou ramifié (par exemple un groupe alkylidène ou alkylène) de 1 à 10 C éventuellement substitué, de préférence sur le même carbone, par un ou plusieurs halogènes choisis parmi F, Cl, Br et I et/ou par un ou plusieurs groupes hydroxyle(s), de préférence encore ledit groupe divalent est un groupe divalent dérivé d'un groupe alkyle perfluoré, par exemple alkylène perfluoré ;
- un hétéroatome choisi parmi O, S ;
- un groupe

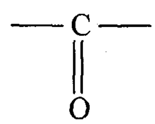
;

un groupe

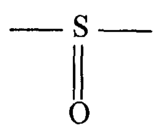
;

un groupe

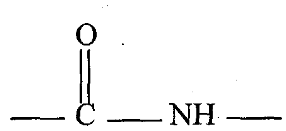
,

un groupe

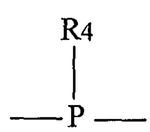
;

un groupe

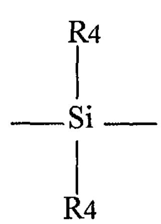
;

un groupe

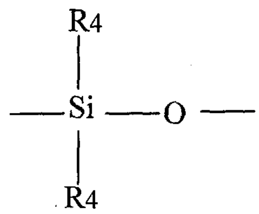

où $R_4$ est choisi parmi les groupes alkyle de 1 à 10 C tels que méthyle, éthyle, isopropyle, etc.

$R_1$ peut également représenter un groupe carboné polycyclique condensé éventuellement substitué par un ou plusieurs substituant(s) choisi(s) parmi les groupes alkyle et les groupes alcoxy de 1 à 10 C, les atomes d'halogène, le groupe nitro et le groupe sulfonyle, ledit groupe carboné polycyclique comprenant par exemple de 2 à 5 cycles benzéniques choisis par exemple parmi le naphtalène, le phénanthrène, le coronène, le pérylène, le phénylindane, etc.

$R_1$ peut aussi représenter un hétérocycle ou un hétérocycle condensé, à caractère aromatique tel que thiophène, pyrazine, pyridine, furanne, quinoléine, quinoxaline, isobenzofuranne, cet hétérocycle étant éventuellement substitué par un ou plusieurs substituants choisis parmi les groupes alkyles (par exemple méthyle, éthyle, isopropyle, etc.) et alcoxy de 1 à 10 C, les atomes d'halogènes (F, Cl, Br, I), le groupe nitro et le groupe sulfonyle.

Parmi les polyimides-amides utilisables dans le cadre de l'invention, on citera ceux dans lesquels $R_1$ est un cycle benzénique, un ensemble de deux cycles benzéniques reliés entre eux par un pont oxygène, ou un cycle naphtalénique.

Les groupes $R_1$ préférés sont :

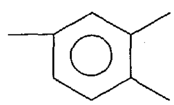

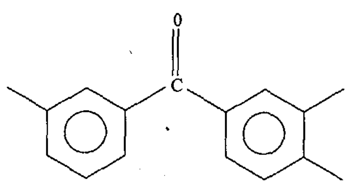

et

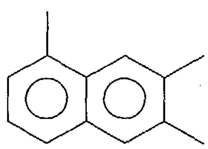

Le groupe $R_3$ répond à la même définition que le groupe $R_1$ à la différence qu'il s'agit d'un groupe tétravalent et non trivalent.

R et $R_2$ qui peuvent être identiques ou différents représentent chacun par exemple un cycle benzénique divalent à enchaînement meta, ou para ; éventuellement substitué par un ou plusieurs substituants choisis parmi les groupes alkyle et alcoxy de 1 à 10 C tels que méthyle, éthyle, isopropyle, butyle, méthoxy...., les atomes d'halogène, le groupe nitro et le groupe sulfonyle ; ou plusieurs cycles benzéniques éventuellement substitués par un ou plusieurs substituants choisis parmi les groupes alkyles et alcoxy de 1 à 10C, les atomes d'halogène, le groupe nitro, et le groupe sulfonyle, par exemple R et $R_2$ peuvent comprendre de 2 à 5 cycles, reliés entre eux par une simple liaison ou par un groupe divalent.

Ledit groupe divalent reliant les cycles benzéniques de R ou $R_2$ est choisi par exemple parmi :

- un groupe divalent dérivé d'un groupe alkyle linéaire ou ramifié (par exemple un groupe alkylidène ou alkylène) de 1 à 10 C éventuellement substitué, de préférence sur le même carbone par un ou plusieurs halogènes choisis parmi F, Cl, Br et I et/ou par un ou plusieurs groupes hydroxyle(s), de préférence encore ledit groupe divalent est un groupe divalent dérivé d'un groupe alkyle perfluoré, par exemple alkylène perfluoré ;
- un hétéroatome choisi parmi O, S ;
- un groupe

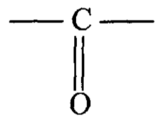 ;

un groupe

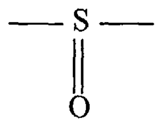 ;

un groupe

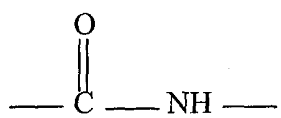 ,

un groupe

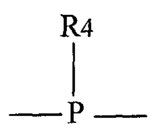 ;

un groupe

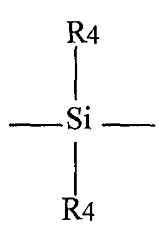

un groupe

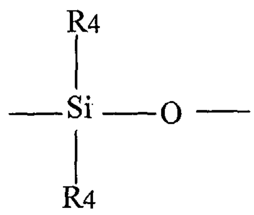

où $R_4$ est choisi parmi les groupes alkyle de 1 à 10 C tels que méthyle, éthyle, isopropyle, etc.

R et $R_2$ peuvent également chacun représenter un groupe carboné polycyclique condensé divalent éventuellement substitué par un ou plusieurs substituant(s) choisi(s) parmi les groupes alkyle et alcoxy de 1 à 10 C, les atomes d'halogène, le groupe nitro et le groupe sulfonyle, ledit groupe carboné polycyclique peut comprendre par exemple de 2 à 5 cycles benzéniques, et il peut être choisi par exemple parmi le naphtalène, le phénanthrène, le coronène, le pérylène, le phénylindane, etc.

$R_2$ peut aussi représenter un hétérocycle ou un hétérocycle condensé à caractère aromatique par exemple thiophène, pyrazine, pyridine, furanne, quinoléine, quinoxaline, isobenzofuranne, cet hétérocycle étant éventuellement substitué par un ou plusieurs substituant(s) choisi(s) parmi les groupes alkyle, et alcoxy de 1 à 10 C, par exemple méthyle, éthyle, isopropyle, méthoxy, les atomes d'halogènes (F, Cl, Br, I), le groupe nitro, et le groupe sulfonyle.

Les polyimides-amides préférées sont ceux dans lesquels R est un groupe diphénylméthane et $R_2$, un groupe phényl-1,4-diyle ; ou R un groupe diphényléther, et $R_2$ est un groupe phényl-1,4-diyle.

Des exemples du groupe $R_1$ ont déjà été donnés plus haut :

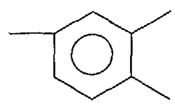

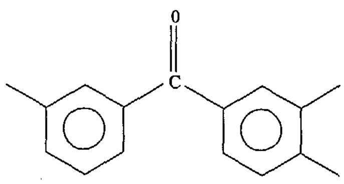

et

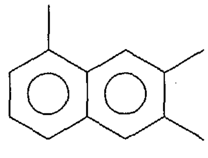

Des exemples du groupe $R_3$ sont les suivants :

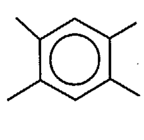
;
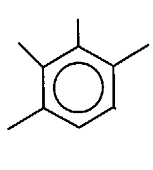
;

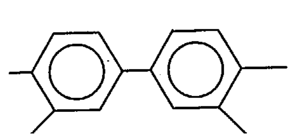 ; 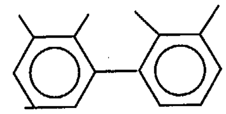 ;

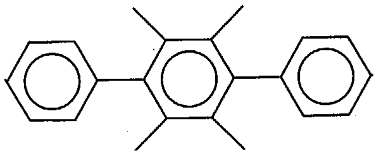 ; 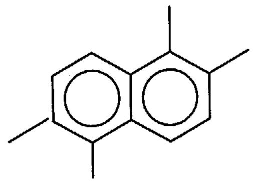 ;

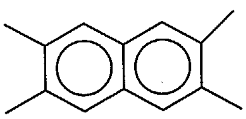 ; 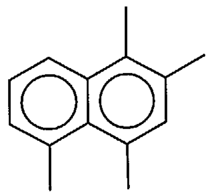 ; 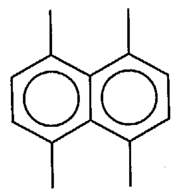 ;

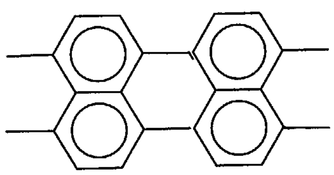 ; 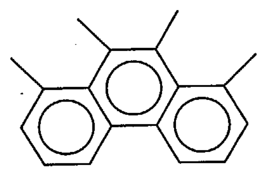 ;

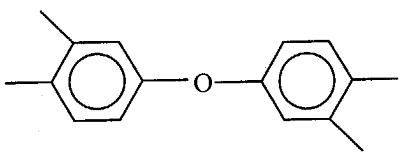 ; 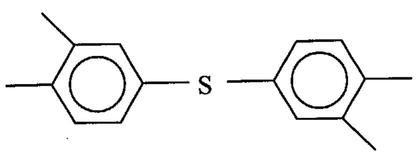

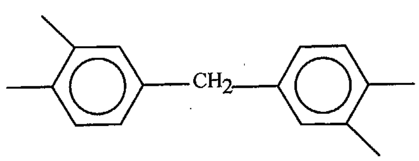 ; 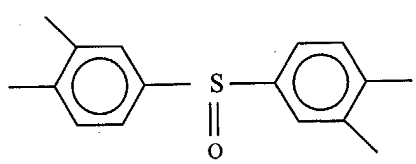 ;

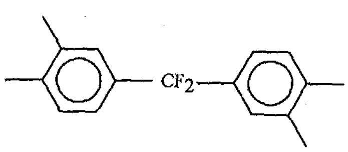 ; 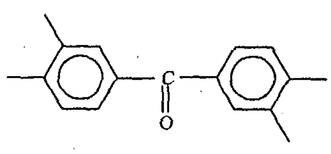 ;

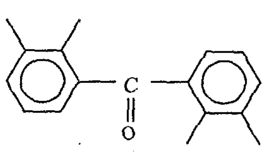 ; 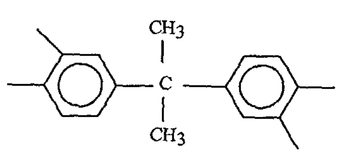 ;

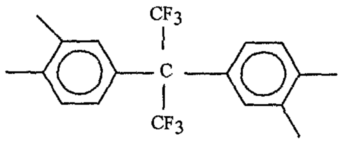 ; 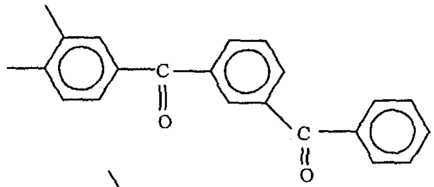 ;

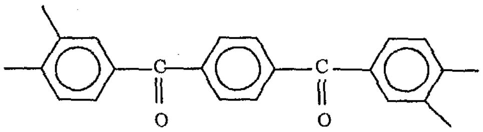 ; 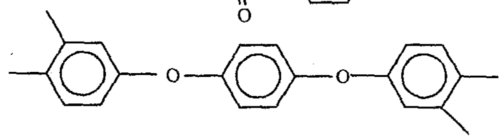 ;

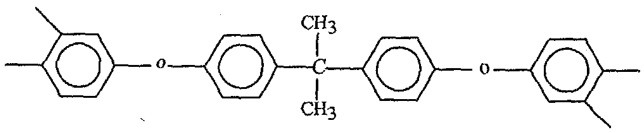 ; 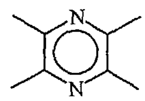 ;

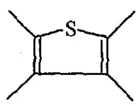 ; 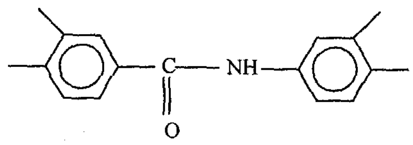

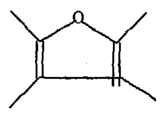

Il est à noter que d'autres exemples de groupes $R_1$ sont les équivalents trivalents des groupes tétravalents $R_3$ exemplifiés ci-dessus.
Des exemples des groupes R et $R_2$ sont les suivants :

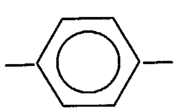 ; 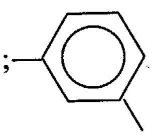 ; 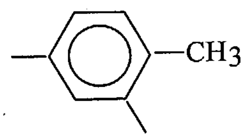 ; 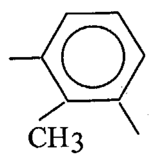 ;

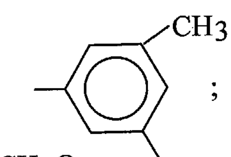 ; 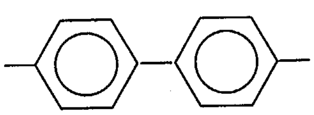 ; 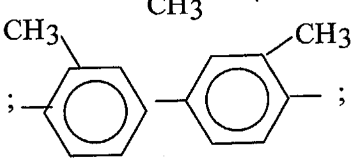 ;

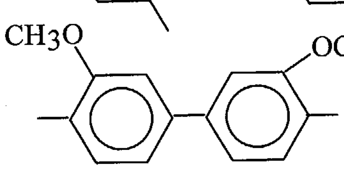 ; 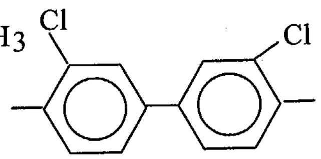 ; 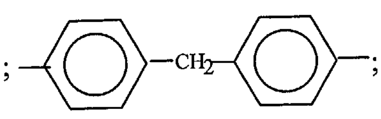 ;

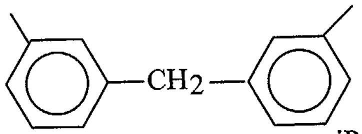 ; 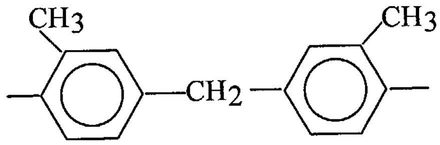 ;

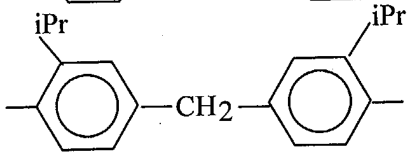 ; 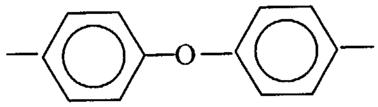 ;

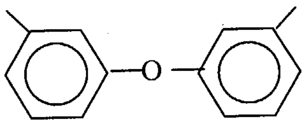 ; 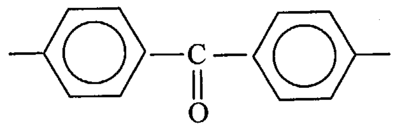 ;

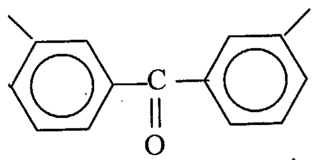 ; 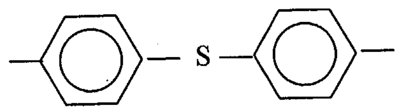 ;

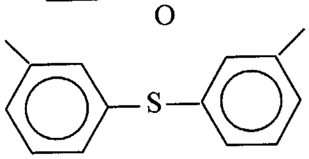 ; 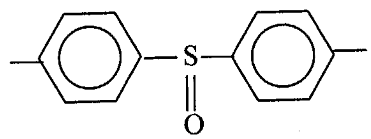 ;

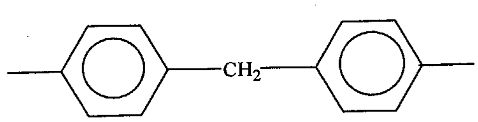 ; 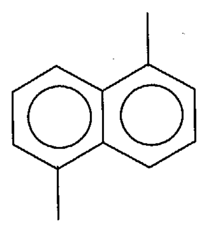

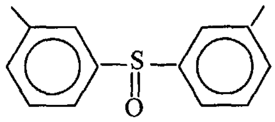 ; 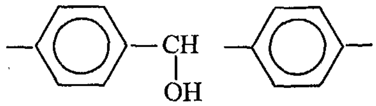 ;

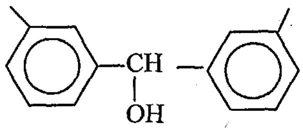 ; 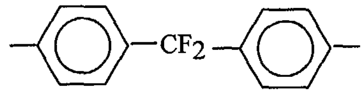 ;

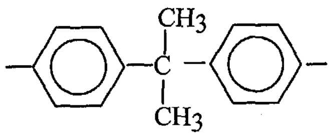 ; 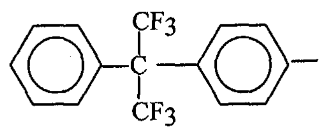 ; 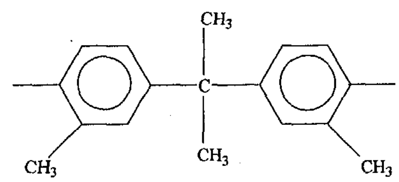

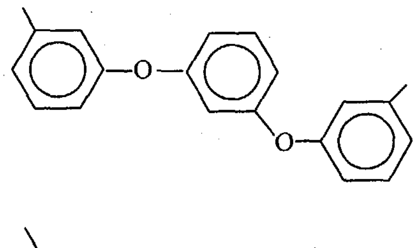 ; 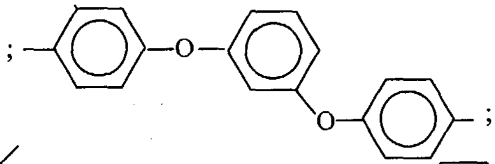 ;

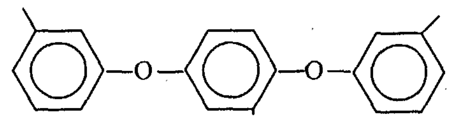 ; 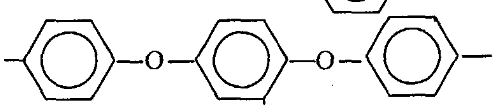 ;

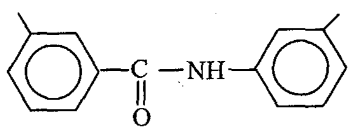 ; 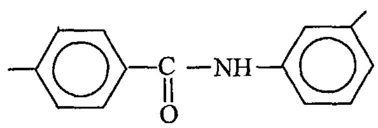 ;

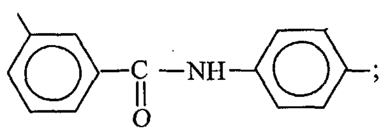 ; 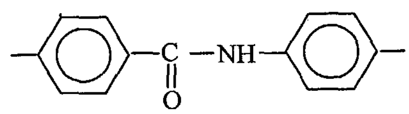 ;

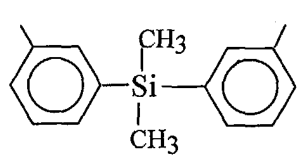 ; 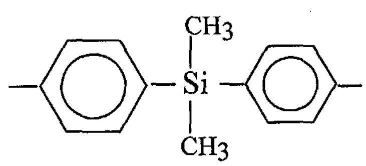 ; 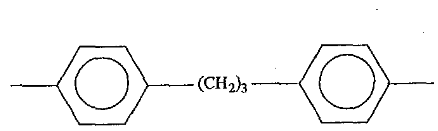

Les polyimides-amides aromatiques formant les fibres mises en oeuvre dans la composition de l'invention peuvent être obtenus par tous les procédés connus de l'homme du métier pour la préparation des polyimides-amides aromatiques, de préférence, par réaction d'un diisocyanate avec l'anhydride triméllitique.

Des fils ou fibres de polyimide-amide, qui peuvent être utilisés pour les compositions de l'invention, sont décrits, par exemple, dans le document de R. PIGEON et P. ALLARD, Chimie Macromoléculaire Appliquée, 40/41 (1974), pages 139-158 (n° 600), ou bien encore dans les documents US-A-3 802 841, FR-A-2 079 785, EP-A1-0 360 728, EP-A-0 549 494, auxquels on pourra se référer,

En vue d'être incorporés dans la composition de l'invention, les filaments sont ensuite découpés en fibres de la longueur voulue, mentionnée plus haut.

Comme on l'a déjà indiqué plus haut, les fibres de polyimide-amide aromatique préférées sont des fibres de

KERMEL TECH®, dans lesquelles le polyimide-amide comprend des motifs répétitif de formule :

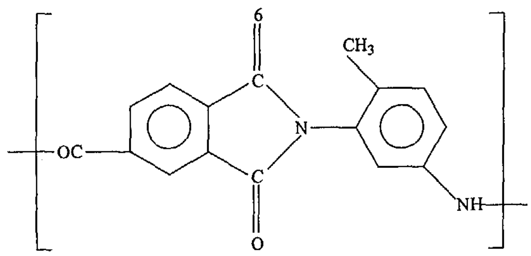

et est obtenu par polycondensation du toluylène diisocyanate et de l'anhydride triméllitique.

Les fibres rigides peuvent être présentes dans la composition selon l'invention en une teneur allant de 0,01 % à 10 % en poids, par rapport au poids total de la composition, de préférence de 0,1 % à 5 % en poids, et mieux de 0,3 % à 3 % en poids.

La composition selon l'invention a un comportement plastique thixotrope.

On entend dans la présente demande par composition ayant un comportement plastique thixotrope, une composition ayant les propriétés suivantes :

- la composition a un caractère rhéofluidifiant, c'est-à-dire que la viscosité de la composition diminue lorsque l'on applique à la composiiton des cisaillements croissants ;
- la composition après l'application d'un cisaillement intense se fluidifie (notamment sa viscosité diminue) mais la déstructuration de la composition est retardée dans le temps. Notamment, la viscosité, la consistance et l'élasticité de la composition après sa déstructuration, en particulier après un temps de repos d'une minute après avoir appliqué le cissailement, sont inférieures à celles de la composition avant l'application du cisaillement intense ;
- la composition régénère sa structure initiale en partie ou en totalité qu'après un temps de repos suffisant. La restructuration de la composition ne se produit donc pas instantanément mais de façon différée dans le temps. Notamment, la composition, soumise à un cisaillement constant de 1000 $s^{-1}$ pendant une minute, retrouve en partie ou en totalité sa viscosité initiale après un temps de repos suffisant qui peut être plus ou moins long.

Une définition de composition thixotrope est notamment indiquée dans l'ouvrage "Comprendre la rhéologie - De la circulation du sang à la prise du béton" de P. Cousot et J.L. Grossiord, EDP Science, 2002, pages 16 et 17.

Le comportement plastique thixotrope de la composition peut être évalué selon le protocole d'évaluation du caractère thixotrope décrit ci-après :
Le comportement plastique thixotrope de la composition peut être caractérisé par la consistance G* , l'élasticité $\delta$ et le seuil d'écoulement $\tau_c$ ; ces paramètres sont notamment définis dans l'ouvrage "Initiation à la rhéologie", G. Couarraze et J.L. Grossiord, 2ème édition, 1991, Edition Lavoisier-Tec 1 Doc.
Ces paramètres sont déterminés par des mesures effectuées à 25 °C ± 0,5 °C à l'aide du rhéomètre à contrainte imposée Haake 75 de la société Thermo Rhéo, équipé d'un mobile en acier inoxydable à géométrie plan/plan, le plan ayant un diamètre de 20 mm et un entrefer (distance entre le plan inférieur - appelé plan stator - sur lequel est déposée la composition et le plan supérieur - appelé plan rotor) de 0,3 mm. Les 2 plans sont striés pour limiter les phénomènes de glissement aux parois des plans.

a) On mesure d'abord en régime oscillatoire les caractéristiques rhéologiques de la composition sous faible cisaillement évitant la déstructuration de la composition (conditions considérées comme permettant d'évaluer les caractéristiques rhéologiques de la composition au repos) en imposant à la composition un cisaillement harmonique selon une contrainte $\tau(t)$ variant de façon sinusoïdale selon une pulsation $\omega$ ($\omega = 2\Pi$ N, N étant la fréquence du cisaillement appliqué). La composition ainsi cisaillée subie une contrainte $\tau(t)$ et répond selon une déformation $\gamma(t)$ correspondant à des microdéformations pour lesquelles la consistance varie peu en fonction de la contrainte imposée.

La contrainte $\tau(t)$ et la déformation $\gamma(t)$ sont définies respectivement par les relations suivantes :

$$\tau(t) = \tau_0 \cos \omega t \qquad \gamma(t) = \gamma_0 \cos(\omega t - \delta)$$

$\tau_0$ étant l'amplitude maximale de la contrainte et $\gamma_0$ étant l'amplitude maximale de la déformation.

Les mesures sont effectuées à une fréquence de 1 Hz (N = 1 Hz).

On mesure ainsi l'évolution de la consistance G* (correspondant au rapport de la contrainte imposée sur la déformation mesurée) et de l'élasticité $\delta$ (correspondant à l'angle de déphasage de la contrainte appliquée par rapport à la déformation mesurée) en fonction de la contrainte $\tau(t)$ appliquée.

On mesure en particulier la déformation de la composition pour la zone de contrainte pour lesquelles la consistance initiale $G^*_i$ et l'élasticité initiale $\delta_i$ varient peu (zone des microdéformations dans laquelle la variation de la consistance initiale et de l'élasticité initiale est inférieure à 15 %) et on détermine ainsi la consistance initiale $G^*_i$

b) Puis on destructure la composition en lui appliquant un cisaillement continu intense $\dot{\gamma}$ de 1000 $s^{-1}$ pendant 60 secondes.

c) Après la destructuration de la composition, on suit au cours du temps de repos la restructuration de la composition en imposant à la composition un cisaillement harmonique en régime oscillatoire selon une très faible contrainte variant de façon sinusoïdale à une fréquence de 1 Hz, la contrainte étant telle que la déformation de la composition correspond à des microdéformations pour lesquelles la consistance de la composition varie peu (la variation de la consistance est inférieure à 15 %) en fonction de cette contrainte appliquée.

On mesure ensuite la consistance G* de la composition en fonction du temps de repos ; on détermine alors la valeur de la consistance après un temps de repos d'une minute ($G_1^*$) et après un temps de repos de 30 minutes ($G_{30}^*$).

On détermine alors la reprise en thixotropie de la composition après x minutes correspondant au rapport : $100 \times (G^*_i - G_x^*)/G_x^*$.

La reprise en thixotropie après 1 minute de repos est calculée avec la valeur $G_1^*$ (x = 1) ; la reprise en thixotropie après 30 minutes est calculée avec la valeur $G_{30}^*$ (x = 30) .

Le comportement plastique thixotrope de la composition est notamment caractérisé par une consistance initiale $G_i^*$ allant de $1 \times 10^2$ Pa à $1 \times 10^5$ Pa, de préférence allant de $5 \times 10^2$ Pa à $5 \times 10^4$ Pa, et mieux allant de $6 \times 10^2$ Pa à $9 \times 10^3$ Pa, mesurée sous une contrainte sinusoïdale à une fréquence de 1 Hz.

En outre, la composition peut présenter une élasticité initiale $\delta_i$ pouvant aller de 1 ° à 45 °, et mieux allant de 10 ° à 35 °.

En particulier; la composition a un seuil d'écoulement $\tau_c$ allant de 10 Pa à 3500 Pa, et de préférence allant de 20 Pa à 1000 Pa, ce qui signifie que la composition selon l'invention ne s'écoule pas sous son propre poids mais qu'il est nécessaire d'appliquer à la composition une contrainte critique à partir de laquelle on provoque son écoulement.

Préférentiellement, la composition, après être soumise à un cisaillement continu de 1000 $s^{-1}$ pendant une minute, présente une reprise en thixotropie,:

- (i) inférieure ou égale à 20 %, de préférence allant de 0,1 % à 20 %, après 1 minute de repos
- (ii) inférieure ou égale à 90 %, de préférence allant de 20 % à 90 %, après 30 minutes de repos.

La composition selon l'invention peut comprendre un milieu aqueux, constituant une phase aqueuse, qui peut être la phase continue de la composition.

La composition peut comprendre de l'eau et éventuellement un solvant organique hydrophile (solvant organique miscible à l'eau) comme les alcools et notamment des monoalcools ayant de 2 à 5 atomes de carbone comme l'éthanol, l'isopropanol ou le n-propanol, les polyols ayant de 2 à 8 atomes de carbone comme la glycérine, la diglycérine, le propylène glycol, l'éthylène glycol, le1,3-butylène glycol, le sorbitol, le penthylène glycol, les cétones en $C_3$-$C_4$, les aldéhydes en $C_2$-$C_4$.

L'eau ou le mélange d'eau et de solvant(s) organique(s) hydrophile(s) peut être présent dans la composition selon l'invention en une teneur allant de 0,1 % à 90 % en poids, par rapport au poids total de la composition, et de préférence de 0,1 % à 60 % en poids.

La composition peut également comprendre un milieu huileux, ou phase grasse liquide, comprenant un corps gras choisis parmi les huiles, les solvants organiques, et leurs mélanges. La phase grasse peut former une phase continue de la composition. En particulier, la composition selon l'invention peut être anhydre.

La phase grasse liquide peut notamment être constituée de toute huile physiologiquement acceptable et en particulier cosmétiquement acceptable, notamment choisie parmi les huiles d'origine minérale, animale, végétale ou synthétique, carbonées, hydrocarbonées, fluorées et/ou siliconées, seules ou en mélange.

La phase grasse liquide totale de la composition peut représenter de 0,1 % à 98 % en poids, par rapport au poids total de la composition, et de préférence de 1 à 80 % en poids.

Avantageusement, la phase grasse liquide de la composition peut comprendre au moins une huile ou solvant organique volatile et/ou au moins une huile non volatile.

Par " huile ou solvant organique volatile", on entend au sens de l'invention tout milieu non aqueux susceptible de s'évaporer au contact de la peau ou la fibre kératinique en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg). Par "huile non volatile", on entend une huile restant sur la peau ou la fibre kératinique à température ambiante et pression atmosphérique au moins plusieurs heures et ayant notamment une pression de vapeur inférieure à $10^{-3}$ mm de Hg (0,13Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en $C_8$-$C_{16}$ le néopentanoate d'isohexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité ≤ 6 centistokes (8 $10^{-6}$ $m^2/s$), et ayant notamment de 2 à 10 atomes de silicium, ces silicones comportant éventuellement des groupes alkyle ou alkoxy ayant de 1 à 22 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également utiliser des solvants volatils fluorés tels que le nonafluorométhoxybutane ou le perfluorométhylcyclopentane.

L'huile volatile peut être présente dans la composition selon l'invention en une teneur allant de 0,1 % à 98 % en poids, par rapport au poids total de la composition, de préférence de 1 % à 65 % en poids.

La composition peut également comprendre au moins une huile non volatile, et notamment choisie parmi les huiles hydrocarbonées et/ou siliconées et/ou fluorées non volatiles.
Comme huile hydrocarbonée non volatile, on peut notamment citer :

- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_5 + R_6$ soit ≥ 10, comme par exemple l'huile de Purcellin (octanoate de cétostéaryle), le myristate d'isopropyle, le palmitate d'isopropyle, le benzoate d'alcool en $C_{12}$ à $C_{15}$, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de diisostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;

- les acides gras supérieurs tels que l'acide oléique, l'acide linoléique, l'acide linolénique ;

et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

L' huile non volatile peut être présente dans la composition selon l'invention en une teneur allant de 0,1 % à 80 % en poids, de préférence de 0,1 % à 50 % en poids, par rapport au poids total de la composition, et mieux de 0,1 % à 20 % en poids.

La composition selon l'invention peut comprendre un agent épaississant thixotrope en une quantité suffisante pour conférer à la composition le comportement plastique thixotrope.

L'agent épaississant thixotrope peut être présent en une teneur allant de 0,5 % à 15 % en poids, par rapport au poids total de la composition, de préférence allant de 1 % à 15 % en poids, préférentiellement allant de 2 % à 10 % en poids, et plus préférentiellement allant de 2 % à 8 % en poids.

L'agent épaississant est choisi selon le milieu de la composition : si la composition comprend un milieu aqueux, on utilise alors un épaississant de milieu aqueux. Si la composition comprend un milieu huileux, on utilise alors un épaississant de milieu huileux. Lorsque la composition est sous forme d'émulsion, l'agent épaississant thixotrope est de préférence présent dans la phase externe de l'émulsion.

L'agent épaississant de milieu aqueux peut être choisi parmi les argiles hydrophiles, la gomme de carraghénane, la silice pyrogénée hydrophile.

On entend par argile hydrophile une argile apte à gonfler dans l'eau ; cette argile gonfle dans l'eau et forme après hydratation une dispersion colloïdale.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence.

Les argiles sont des silicates contenant un cation pouvant être choisi parmi les cations de calcium, de magnésium, d'aluminium, de sodium, de potassium, de lithium et leurs mélanges.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.

Ces argiles peuvent être d'origine naturelle ou synthétique. De préférence, on utilise les argiles qui sont cosmétiquement compatibles et acceptables avec les matières kératiniques comme les cils, la peau.

Comme argile hydrophile , on peut citer les smectites telles que les saponites, les hectorites, les montmorillonites, les bentonites, la beidellite.

Comme argile hydrophile, on peut citer les hectorites synthétiques (appelées aussi laponites) comme les produits vendus par la société Laporte sous le nom Laponite XLG, Laponite RD, Laponite RDS (ces produits sont des silicates de sodium et de magnésium et en particulier des silicates de sodium, de lithium et de magnésium) ; les bentonites comme le produit vendu sous la dénomination Bentone HC par la société RHEOX ; les silicates de magnésium et d'aluminium notamment hydratés comme les produits vendus par la société Vanderbilt Company sous le nom Veegum ultra, Veegum HS, Veegum DGT, ou encore les silicates de calcium et notamment celui sous forme synthétique vendu par la société sous le nom de Microcel C.

Les silices pyrogénées hydrophiles peuvent être obtenues par hydrolyse à haute température d'un composé volatil du silicium dans une flamme oxhydrique, produisant une silice finement divisée. Les silices hydrophiles présentent un nombre important de groupements silanol à leurs surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

La silice pyrogénée hydrophile présente de préférence une taille de particules pouvant être nanométrique à micrométrique, par exemple allant d'environ de 5 à 200 nm.

L'agent épaississant thixotrope de milieu huileux peut être choisi parmi les argiles organophiles, les silices pyrogénées hydrophobes, les organopolysiloxanes élastomériques.

Les argiles organophiles sont des argiles modifiées par des composés chimiques rendant l'argile apte à gonfler dans les milieux huileux.

L'argile peut être choisie parmi la montmorrilonite, la bentonite, l'hectorite, l'attapulgite, la sépiolite, et leurs

mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :

- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6$^{ème}$ édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6$^{ème}$ édition, 1995). Elles sont par exemple commercialisées sous les références "AEROSIL R972® ", "AEROSIL R974® " par la société Degussa, "CAB-O-SIL TS-610® ", "CAB-O-SIL TS-720® " par la société Cabot.

La silice pyrogénée hydrophobe présente de préférence une taille de particules pouvant être nanométrique à micrométrique, par exemple allant d'environ de 5 à 200 nm.

Les organopolysiloxanes élastomériques sont en général partiellement ou totalement réticulés et éventuellement de structure tridimensionnelle. Inclus dans une phase grasse, ils se transforment, selon le taux de phase grasse utilisé, d'un produit d'aspect spongieux lorsqu'ils sont utilisés en présence de faibles teneurs en phase grasse en un gel homogène, en présence de quantités de phase grasse plus élevées.

Les organopolysiloxanes élastomères associés à une phase grasse se présentent généralement sous forme de gel constitué d'un organopolysiloxane élastomère associé à une phase grasse, inclus dans au moins une huile hydrocarbonée et/ou une huile siliconée. Ils peuvent être choisis notamment parmi les polymères réticulés décrits dans la demande EP-A-0295886.

Selon cette demande, les organopolysiloxanes élastomères sont obtenus par réaction d'addition et de réticulation d'au moins :

(a) un organopolysiloxane ayant au moins deux groupes alcényle inférieurs par molécule ;
(b) un organopolysiloxane ayant au moins deux atomes d'hydrogène liés à un atome de silicium par molécule ; et
(c) et un catalyseur du type platine.

Les groupes alcényle inférieurs sont notamment les groupes vinyl, allyl et propényl.
Le catalyseur au platine peut être par exemple l'acide chloroplatinique, les complexes contenant de l'acide chloroplatinique et le platine supporté par un support approprié.

Les organopolysiloxanes élastomériques associés à une phase grasse peuvent être choisis aussi parmi ceux décrits dans le brevet US-A-5266321.

Selon ce brevet, ils sont choisis notamment parmi :

i) les polyorganopolysiloxanes comprenant des motifs $R_2SiO$ et $RSiO_{1,5}$ et éventuellement des motifs $R_3SiO_{0,5}$ et/ou $SiO_2$ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel que vinyle et où le rapport en poids des motifs $R_2SiO$ sur les motifs $RSiO_{1,5}$ varie de 1/1 à 30/1 ;

ii) les polyorganopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20 % mol quand l'organopolysiloxane est non-cyclique et entre 1 et 50 % mol lorsque l'organopolysiloxane est cyclique.

La composition selon l'invention peut comprendre, en outre, un agent épaississant additionnel différent des agents épaississants thixotropes décrits précédemment.

Lorsque la composition selon l'invention comprend un milieu aqueux, elle peut donc comprendre un agent épaississant additionnel de milieu aqueux. Cet agent épaississant n'est pas apte à lui seul à rendre à la composition le caractère plastique thixotrope (épaississant non thixotrope) ; il permet notamment d'ajuster la viscosité de la composition pour obtenir un écoulement homogène.

Lorsque la composition comprend un milieux aqueux, l'agent épaississant additionnel est alors choisi parmi les agents épaississants hydrophiles (agent épaisissant non thixotrope de milieux aqueux).

Parmi les agents épaississants hydrophiles additionnels utilisables selon l'invention, on peut citer :

- les épaississants cellulosiques hydrosolubles tels que l'hydroxyéthylcellulose, la méthylcellulose, l'hydroxypropylcellulose et la carboxyméthylcellulose. Parmi celles-ci, on peut citer notamment les gommes vendues sous la dénomination de "Cellosize QP 4400 H" par la Société Amercol,
- la gomme de guar, notamment celles vendues sous la dénomination VIDOGUM GH 175 par la société UNIPECTINE et sous la dénomination JAGUAR C par la société MEYHALL,
- la gomme de guar quaternisée vendue sous la dénomination de "Jaguar C-13-S" par la Société Meyhall,
- les gommes de guar non-ioniques comprenant des groupements hydroxyalkyle en $C_1$-$C_6$. On peut mentionner à titre d'exemple, les groupements hydroxyméthyle, hydroxyéthyle, hydroxypropyle et hydroxybutyle. De telles gommes de guar sont notamment vendues sous les dénominations commerciales JAGUAR HP8, JAGUAR HP60 et JAGUAR HP120 et JAGUAR HP 105 par la société MEYHALL, ou sous la dénomination GALACTASOL 40H4FD2 par la société AQUALON.
- les gommes de xanthane, de caroube, de scléroglucane, de gellane, de rhamsan, de karaya,
- les alginates, la maltodextrine, l'amidon et ses dérivés, l'acide hyaluronique et ses sels,
- les polymères poly(métha)crylates de glycéryle vendus sous les dénominations de "Hispagel" ou "Lubragel" par les Sociétés Hispano Quimica ou Guardian,
- la polyvinylpyrrolidone,
- l'alcool polyvinylique,
- les polymères et les copolymères réticulés d'acrylamide, tels que ceux vendus sous les dénominations de "PAS 5161" ou "Bozepol C" par la Société Hoechst, de "Sepigel 305" par la Société Seppic par la Société Allied Colloïd, ou encore
- les homopolymères réticulés de chlorure de méthacryloyloxyéthyltriméthylammonium vendus sous la dénomination de "Salcare SC95" par la Société Allied Colloïd.
- les polymères associatifs et notamment les polyuréthanes associatifs.

L'agent épaississant additionnel hydrophile est de préférence un polyuréthane associatif.
Les polyuréthannes associatifs sont des copolymères séquences non ioniques comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

En particulier, ces polymères comportent au moins deux chaînes lipophiles hydrocarbonées, ayant de $C_6$ à $C_{30}$ atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polymères peuvent être séquences sous forme de tribloc ou multibloc. Les séquences hydrophobes peuvent donc être à chaque extrémité de la chaîne (par exemple : copolymère tribloc à séquence centrale hydrophile) ou réparties à la fois aux extrémités et dans la chaîne (copolymère multiséquencé par exemple). Les polymères peuvent être également en greffons ou en étoile.

De préférence, les polymères sont des copolymères triblocs dont la séquence hydrophile est une chaîne polyoxyéthylénée comportant de 50 à 1 000 groupements oxyéthylénés. En général les polyuréthannes associatifs comportent une liaison uréthane entre les séquences hydrophiles, d'où l'origine du nom.

A titre d'exemple, des polymères associatifs utilisables dans l'invention, on peut citer le polymère $C_{16}$-$OE_{120}$-$C_{16}$ vendu par la société HULS (sous le nom Sérad FX1100, molécule à fonction uréthanne et poids moléculaire moyen en poids de 1300), OE étant un motif oxyéthyléné. Comme polymère associatif, on peut aussi utiliser aussi le Rhéolate 205 à fonction urée vendu par la société RHEOX ou encore le Rhéolate 208 ou 204. Ces polyuréthannes associatifs sont vendus sous forme pure.

Le produit DW 1206B de chez RHOM & HAAS à chaîne alkyle en $C_{20}$ et à liaison uréthane, vendu à 20 % en matière sèche dans l'eau, peut aussi être utilisé.

On peut aussi utiliser des solutions ou dispersions de ces polymères notamment dans l'eau ou en milieu

hydroalcoolique. A titre d'exemple, de tels polymères on peut citer, le Sérad FX1010, le Sérad FX1035 et le Serad 1070 vendus par la société HULS, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS, ou bien encore le Borchigel LW 44 de la société BORCHERS.

Les polymères utilisables dans l'invention sont en particulier ceux décrits dans l'article de G. Fonnum, J. Bakke et Fk. Hansen - Colloid Polym. Sci 271, 380.389 (1993).

Lorsque la composition comprend un milieu huileux, l'agent épaississant additionnel est alors choisi parmi les agents épaississants lipophiles (agent épaississant non thixotrope de milieux huileux).

Parmi les agents épaississants lipophiles additionnels utilisables selon l'invention, on peut citer :

- les gommes de guar alkylées (avec groupe alkyle en $C_1$-$C_6$), telles que celles décrites dans EP-A-708114 ;
- les polymères gélifiant d'huile comme les polymères tribloc ou en étoile résultant de la polymérisation ou copolymérisation d'au moins monomère à groupe éthylénique, comme les polymères vendus sous la dénomination Kraton ;
- les résines de polyamides comprenant des groupes alkyles ayant de 12 à 22 atomes de carbone, telles que celles décrites dans US-A-5783657.

L'agent épaississant additionnel peut être présent en une teneur allant de 0,1 % à 5 % en poids, par rapport au poids total de la composition, et de préférence allant de 0,1 % à 3 % en poids.

La composition selon l'invention comprend au moins un composé choisi parmi un polymère filmogène et/ou une cire.

Le polymère filmogène peut être un polymère solubilisé ou dispersé sous forme de particules dans une phase aqueuse de la composition ou bien encore solubilisé ou dispersé sous forme de particules dans une phase grasse liquide. La composition peut comprendre un mélange de ces polymères.

Le polymère filmogène peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Dans la présente demande, on entend par "polymère filmogène", un polymère apte à former à lui seul ou en présence d'un agent auxiliaire de filmification, un film continu et adhérent sur un support, notamment sur les matières kératiniques.

On utilise de préférence un polymère filmogène apte à former un film hydrophobe, c'est-à-dire un polymère dont le film a une solubilité dans l'eau à 25 °C inférieure à 1 % en poids.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide. (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$ .

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire

qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

Il est possible d'utiliser tout monomère connu de l'homme du métier entrant dans les catégories de monomères acryliques et vinyliques (y compris les monomères modifiés par une chaîne siliconée).

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyétherpolyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol.

Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement $-SO_3M$, avec M représentant un atome d'hydrogène, un ion ammonium $NH_4^+$ ou un ion métallique, comme par exemple un ion $Na^+$, $Li^+$, K+, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement $-SO_3M$

Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement $-SO_3M$ : l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ® par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le polymère filmogène peut être présent sous la forme de particules en dispersion aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90®, NEOCRYL A-1070®, NEOCRYL A-1090®, NEOCRYL BT-62®, NEOCRYL A-10790®, NEOCRYL A-523® par la société AVECIA-NEORESINS, DOW LATEX 432® par la société DOW CHEMICAL, DAITOSOL 5000 AD® par la société DAITO KASEY KOGYO; ou ben encore les dispersions aqueuses de polyuréthane

vendues sous les dénominations NEOREZ R-981®, NEOREZ R-974® par la société AVECIA-NEORESINS, les AVALURE UR-405®, AVALURE UR-410®, AVALURE UR-425 ®, AVALURE UR-450®, SANCURE 875®, SANCURE 861®, SANCURE 878®, SANCURE 2060® par la société GOODRICH, IMPRANIL 85® par la société BAYER, AQUAMERE H-1511® par la société HYDROMER.

Comme dispersion aqueuse de polymère filmogène, on peut également utiliser les dispersions de polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

Selon une deuxième variante de réalisation de la composition selon l'invention, le polymère filmogène peut être un polymère hydrosoluble et est donc présent dans la phase aqueuse de la composition sous forme solubilisée. Comme exemples de polymères filmogènes hydrosolubles, on peut citer

- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :
  - les gommes arabiques, la gomme de guar, les dérivés du xanthane, la gomme de karaya ;
  - les alginates et les carraghénanes ;
  - les glycoaminoglycanes, l'acide hyaluronique et ses dérivés ;
  - la résine shellac, la gomme de sandaraque, les dammars, les élémis, les copals ;
  - l'acide désoxyribonucléïque ;
  - les muccopolysaccharides tels que l'acide hyaluronique, les chondroïtines sulfate, et leurs mélanges.

Selon une autre variante de réalisation de la composition selon l'invention, le polymère filmogène peut être présent dans une phase grasse liquide comprenant des huiles ou solvants organiques tels que ceux décrits précédemment. Par "phase grasse liquide", on entend, au sens de l'invention, une phase grasse liquide à température ambiante (25°C) et pression atmosphérique (760 mm de Hg, soit 105 Pa), composée d'un ou plusieurs corps gras liquides à température ambiante, appelés aussi huiles, généralement compatibles entre eux.

De préférence, la phase grasse liquide comprend une huile volatile, éventuellement en mélange avec une huile non volatile, les huiles pouvant être choisies parmi les huiles citées précédemment.

Selon un troisième mode de réalisation de la composition selon l'invention, le polymère filmogène peut être présent sous forme de particules, stabilisées en surface, dispersées dans la phase grasse liquide.

La dispersion de particules de polymère stabilisées en surface peut être fabriquée comme décrit dans le document EP-A-749747.

Les particules de polymère sont stabilisées en surface grâce à un stabilisant qui peut être un polymère séquence, un polymère greffé, et/ou un polymère statistique, seul ou en mélange.

Des dispersions de polymère filmogène dans la phase grasse liquide, en présence d'agent stabilisants, sont notamment décrites dans les documents EP-A-749746, EP-A-923928, EP-A-930060 dont le contenu est incorporé à titre de référence dans la présente demande.

La taille des particules de polymères en dispersion soit dans la phase aqueuse, soit dans la phase grasse liquide, peut aller de 5 nm à 600 nm, et de préférence de 20 nm à 300 nm.

Selon un quatrième mode de réalisation de la composition selon l'invention, le polymère filmogène peut être solubilisé dans la phase grasse liquide, on dit alors que le polymère filmogène est un polymère liposoluble.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 10 à 20 atomes de carbone.

De tels homopolymères liposolubles peuvent être choisis parmi le polystéarate de vinyle, le polystéarate de vinyle réticulé à l'aide de divinylbenzène, de diallyléther ou de phtalate de diallyle, le poly(méth)acrylate de stéaryle, le polylaurate de vinyle, le poly(méth)acrylate de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

La composition selon l'invention peut comprendre un agent auxiliaire de filmification favorisant la formation d'un film avec le polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition selon l'invention peut comprendre une cire.

Un objet de l'invention est donc une composition comprenant, dans un milieu physiologiquement acceptable, des fibres rigides sensiblement rectilignes et une cire, la composition présentant un comportement plastique thixotrope.

Par "cire", on entend au sens de la présente invention, un composé gras lipophile, solide à température ambiante (25°C) et pression atmosphérique (760 mm de Hg, soit 105 Pa), à changement d'état solide/liquide réversible, ayant une température de fusion supérieure à 30°C et mieux supérieure à 55 °C et pouvant aller jusqu'à 200° C, notamment jusqu'à 120 °C.

En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER , avec une montée en température de 5 ou 10 °C par minute.

Les cires, au sens de l'invention, sont celles généralement utilisées dans les domaines cosmétique et dermatologique . On peut notamment citer la cire d'abeilles, la cire de lanoline, et les cires d'insectes de Chine; la cire de riz, la cire de Carnauba, la cire de Candellila, la cire d'Ouricury, la cire de fibres de liège, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40°C et mieux à plus de 55°C.

On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée.

On peut encore citer les cires de silicone ou les cires fluorées.
On peut utiliser un mélange des cires décrites précédemment.

Les cires présentes dans la composition peuvent être dispersées sous forme de particules dans un milieu aqueux. Ces particules peuvent avoir une taille moyenne allant de 50 nm à 50 µm, et de préférence allant de 50 nm à 10 µm, et mieux allant de 50 nm à 3,5 µm.
En particulier, la cire peut être présente sous forme d'émulsion cires-dans-eau, les cires pouvant être sous forme de particules de taille moyenne allant de 1 µm à 50 µm, de préférence allant de 1 µm à 50 µm, et mieux allant de 1 µm à 5 µm.
Dans un autre mode de réalisation de la composition selon l'invention, la cire peut être présente sous forme de microdispersion de cire, la cire étant sous forme de particules dont la taille moyenne est inférieure à 1 µm, et va notamment de 50 nm à 500 nm. Des microdispersions de cires sont décrites dans les documents EP-A-557196, EP-A-1048282.

La cire peut également présenter une dureté allant de 0,05 MPa à 15 MPa, et de préférence allant de 6 MPa à 15 MPa . La dureté est déterminée par la mesure de la force en compression mesurée à 20 °C à l'aide du texturomètre vendu sous la dénomination TA-XT2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

La cire peut être présente dans la composition selon l'invention en une teneur allant de 0,1 % à 50 % en poids, par rapport au poids total de la composition, de préférence de 0,5 % à 30 % en poids, et mieux de 1 % à 20 % en poids.

La composition selon l'invention peut comprendre au moins un composé gras pâteux à température ambiante. Par "corps gras pâteux" au sens de l'invention, on entend des corps gras ayant un point de fusion allant de 25 à 60°C, de préférence de 30 à 45°C et/ou une dureté allant de 0,001 à 0,5 MPa, de préférence de 0,005 à 0,4 MPa.
Les valeurs de point de fusion correspondent au point de fusion mesuré à l'aide d'un calorimètre à balayage différentiel (D. S. C), tel que le calorimètre vendu sous la dénomination DSC 2920 par la société TA Instruments, avec une montée en température de 5 ou 10°C par minute. (Le point de fusion considéré est le point correspondant à la température du pic le plus endotherme du thermogramme).
La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1mm/s puis à une vitesse de mesure de 0,1 mm/s, la profondeur de pénétration étant de 0,3 mm. La valeur relevée de la dureté est celle du pic maximum de la force appliquée.

De préférence, ces corps gras sont des composés hydrocarbonés, éventuellement de type polymérique ; ils peuvent également être choisis parmi les composés siliconés et/ou fluorés ; ils peuvent aussi se présenter sous forme d'un mélange de composés hydrocarbonés et/ou siliconés et/ou fluorés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges. Comme triglycérides d'origine végétale, on peut utiliser les dérivés d'huile de ricin hydrogénée, tels que le "THIXINR" de Rhéox.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stearyl dimethicones notamment ceux vendus par la société Dow Corning sous les noms commerciaux de DC2503 et DC25514, et leurs mélanges.

Le corps gras pâteux peut être présent dans la composition selon l'invention en une teneur allant de 0 à 60% (notamment 0,01 % à 60 %) en poids, par rapport au poids total de la composition, de préférence allant de 0,5 à 45 % en poids, et mieux allant de 2 % à 30 % en poids, dans la composition.

La composition selon l'invention peut contenir des agents tensioactifs émulsionnants présents notamment en une proportion allant de 2 à 30 % en poids par rapport au poids total de la composition, et mieux de 5 % à 15 %.

Ces agents tensioactifs peuvent être choisis parmi des agents tensioactifs anioniques, non ioniques, ou amphotères. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :

- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de $C_1$-$C_6$ alkyl glucose polyoxyéthylénés, les mono-, di-, tri- ou sesqui-oléates ou stéarates de sorbitol ou de glycérol, les laurates de glycérol ou de polyéthylène glycol ; les alkyl ou alkoxy diméthicones copolyols à chaîne alkyle ou alkoxy pendante ou en bout de squelette siliconé ayant par exemple de 6 à 22 atomes de carbone, les esters d'acides gras de polyéthylène glycol (monostéarate ou monolaurate de polyéthylène glycol) ; les esters d'acides gras (stéarate, oléate) de sorbitol polyoxyéthylénés ; les alkyl (lauryl, cétyl, stéaryl, octyl) éthers polyoxyéthylénés et les diméthicones copolyols, et leurs mélanges.
- parmi les tensioactifs anioniques : les acides gras en $C_{16}$-$C_{30}$ neutralisés par les amines, l'ammoniaque ou les sels alcalins, et leurs mélanges.

On utilise de préférence des tensioactifs permettant l'obtention d'émulsion huile-dans-eau ou cire-dans-eau.

La composition peut comprendre des fibres courtes additionnelles, différentes des fibres rigides décrites précédemment et ayant notamment une longueur inférieure à 0,8 mm , notament allant de 0,1 mm à 0,5 mm.

Les fibres courtes peuvent être choisies parmi les fibres de soie, de coton, de laine, de lin, de cellulose extraites notamment du bois, des légumes ou des algues, de polyamide (Nylon® ), de cellulose modifiée (rayonne, viscose, acétate notamment d'acétate de rayonne), en acrylique notamment de polyméthacrylate de méthyle ou de poly 2-hydroxyéthyl méthacrylate; de polyoléfine et notamment de polyéthylène ou de polypropylène, de Téflon® , de collagène insoluble, de polyesters, de polychlorure de vinyle ou de vinylidène, d'alcool polyvinylique, de polyacrylonitrile, de chitosane, de polyuréthane, de polyéthylène phtalate,.

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène.

La composition selon l'invention peut comprendre en outre des charges. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique ( par exemple feuillet, cubique, hexagonale, orthorombique, etc) . On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, les microbilles de polyméthacrylate de méthyle, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les charges peuvent être présentes à raison de 0,01 à 30 % en poids, et de préférence 0,5 % à 15 % en poids.
La composition de l'invention peut comprendre, en outre, tout additif usuellement utilisé en cosmétique tels que les antioxydants, les conservateurs, les parfums, les neutralisants, les actifs cosmétiques ou dermatologiques comme par exemple des émollients, des hydratants, des vitamines, des filtres solaires, et leurs mélanges. Ces additifs peuvent être présents dans la composition en une teneur allant de 0,01 à 20% du poids total de la composition et mieux allant de 0,01 à 10%.
Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.
La composition selon l'invention peut être fabriquée par les procédés connus, généralement utilisés dans le domaine cosmétique ou dermatologique.
L'invention est illustrée plus en détail dans les exemples suivants.

**Exemple 1 :**

On a préparé un mascara ayant la composition suivante :

- Fibres de polyimide-amide de 2 mm de longueur vendue sous la dénomination KERMEL TECH par la société Rhodia 0,63 g
- Saponite (Veegum DGT de la société Vanderbilt) 2,63 g
- Polyuréthane associatif (Ser Ad FX 1100 de la société Servo) 2 g
- Sulfopolyester (AQ 55 S de la société Eastman Chemical) 3 g
- Cire d'abeille 8 g
- Copolymère polyvinylpyrrolidone/1-eicosène (Antaron V 220F de la société ISP) 3 g
- Microbille de copolymère de méthacrylate de méthyle/diméthacrylate d'éthylèneglycol 4 g
- Fibres de polyamide 0,3 mm de long et 0,9 Dtex de la société Paul Bonte 2 g
- Propylène glycol 8 g
- Oxyde de fer noir 8 g
- Conservateurs qs
- Eau qsp 100 g

Ce mascara a le profil rhéologique suivant :

- consistance initiale $G_i$* égale à 10 000 Pa
- élasticité initiale $\delta_i$ égale à 26 °
- seuil d'écoulement $\tau_c$ égal à 100 Pa ;
- reprise en thixotropie, après un cisaillement continu de 1000 $s^{-1}$, pendant une minute égale à 32 % après 1 minute de repos et égale à 84,5 % après 30 minutes de repos.

Les fibres rigides de polyimide-amide sont dispersées de façon homogène dans le mascara. Ce dernier s'applique facilement sur les cils et permet d'obtenir un allongement optimal des cils, les fibres rigides étant fixées dans le prolongement gement optimal des cils, les fibres rigides étant fixées dans le prolongement des cils.

**Exemple 2** :

On prépare un mascara ayant la composition suivante :

- Fibres de polyimide-amide de 2 mm de longueur vendues sous la dénomination KERMEL TECH par la société Rhodia 1 g
- Hectorite modifiée par le chlorure de di-stéaryl diméthyl ammonium vendu sous la dénomination Bentone 38V par la société Elementis 11 g
- Carbonate de propylène 2 g
- Copolymère acétate de vinyle/stéarate d'allyle (65/35) (Mexomère PQ de CHIMEX) 4,5 g
- Polylaurate de vinyle (Mexomère PP de CHIMEX) 1,5 g
- Oxyde de fer noir 7 g
- Conservateur qs
- Isododécane qsp 100 g

Le mascara obtenu a un comportement plastique thixotrope ; il s'applique facilement sur les cils et confère à ces derniers un effet d'allongement homogène et situé dans le prolongement des cils.

**Exemple 3 :**

On prépare un mascara ayant la composition suivante :

- Fibres de polyimide-amide de 2 mm de longueur vendues sous la dénomination KERMEL TECH par la société Rhodia 1 g
- Hectorite modifiée par le chlorure de di-stéaryl diméthyl ammonium vendu sous la dénomination Bentone 38V par la société Elementis 11 g
- Carbonate de propylène 2 g
- Cire de carnauba 8 g
- Oxyde de fer noir 7 g
- Conservateur qs
- Isododécane qsp 100 g

Le mascara obtenu a un comportement plastique thixotrope ; il s'applique facilement sur les cils et confère à ces derniers un effet d'allongement homogène et situé dans le prolongement des cils.

**Revendications**

1. Composition comprenant, dans un milieu physiologiquement acceptable, des fibres rigides sensiblement rectilignes et au moins un composé choisi parmi un polymère filmogène et/ou une cire, la composition présentant un comportement plastique thixotrope.

2. Composition selon la revendication 1, **caractérisée par le fait qu'**au moins 50 % en nombre, de préférence au moins 75 % en nombre, et mieux au moins 90 % en nombre des fibres sont telles que l'angle formé entre la tangente à l'axe central longitudinal de la fibre à l'une des extrémités de la fibre et la droite reliant ladite extrémité au point sur l'axe central longitudinal de la fibre correspondant à la moitié de la longueur de la fibre soit inférieur à 15 ° et l'angle formé entre la tangente à l'axe central longitudinal de la fibre en un point situé à mi-longueur de la fibre et la droite reliant l'une des extrémités au point sur l'axe central longitudinal de la fibre correspondant à la moitié de la longueur de fibre soit inférieur ou égal à 15 °, pour une même longueur de fibre allant de 0,8 mm à 5 mm, de préférence allant de 1 mm à 4 mm, de préférence allant de 1 mm à 3 mm, et mieux de 2 mm.

3. Composition selon la revendication 2, **caractérisée par le fait que** ledit angle est inférieur ou égal à 10°, de préférence inférieur ou égal à 5°.

4. Composition selon l'une quelconque des revendications précédentes, **caractérisée par le fait que** les fibres ont une longueur (L) allant de 0,8 mm à 5 mm , de préférence allant de 1 mm à 4 mm, et préférentiellement allant de 1 mm à 3 mm.

5. Composition selon l'une quelconque des revendications précédentes, **caractérisée par le fait que** les fibres ont une section comprise dans un cercle de diamètre (D) allant de de 2 nm à 500 µm, de préférence allant de 100 nm à 100 µm, et préférentiellement allant de 1 µm à 50 µm.

6. Composition selon l'une quelconque des revendications précédentes, **caractérisée par le fait que** les fibres ont un facteur de forme (L/D) allant de 3,5 à 2 500, de préférence de 5 à 500, et mieux de 5 à 150.

7. Composition selon l'une quelconque des revendications précédentes, **caractérisée par le fait que** les fibres ont un titre allant de 0,15 à 30 deniers, et de préférence allant de 0,18 à 18 deniers.

8. Composition selon l'une quelconque des revendications précédentes, **caractérisée par le fait que** les fibres rigides sensiblement rectilignes sont des fibres d'un polymère synthétique choisi parmi les polyesters, les polyuréthanes, les polymères acryliques, les polyoléfines, les polyamides.

9. Composition selon l'une des revendications précédentes, **caractérisée en ce que** les fibres rigides ne comprennent pas plusieurs couches altérnées de polymères ayant des indices de réfraction différents.

**10.** Composition selon l'une quelconque des revendications précédentes, **caractérisée par le fait que** les fibres rigides sensiblement rectilignes sont des fibres de polyimide-amide aromatique.

**11.** Composition selon l'une quelconque des revendications précédentes, **caractérisée par le fait que** les fibres rigides sensiblement rectilignes sont des fibres de polyimide-amide aromatique, ledit polyimide-amide aromatique comprenant un motif récurrent de formule (1) :

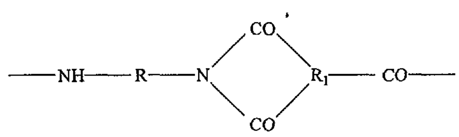

(I)

éventuellement, en outre, un motif récurrent de formule (II) :

$$-NH-R-NH-CO-R_2-CO- \qquad (II)$$

éventuellement, en outre, un motif récurrent de formule (III) :

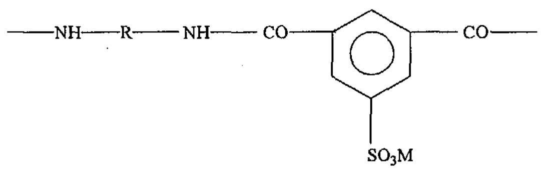

(III)

éventuellement, en outre, un motif récurrent de formule (IV) :

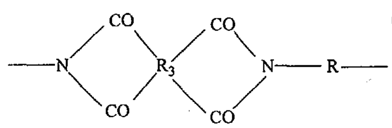

(IV)

**12.** Composition selon la revendication 11, **caractérisée par le fait que** $R_1$ reprsente : un groupe

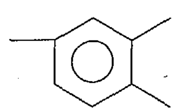

,

un groupe

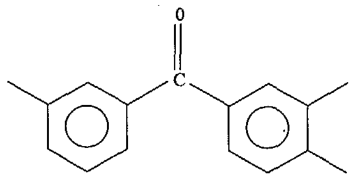 ,

ou un groupe

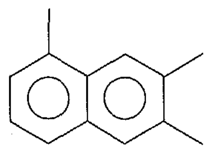 .

**13.** Composition selon l'une des revendications 11 ou 12, **caractérisée par le fait que** R est choisi parmi les groupes :

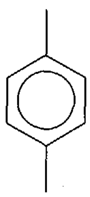 ; 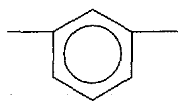 ; 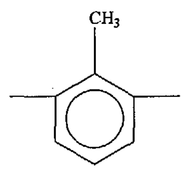 ; 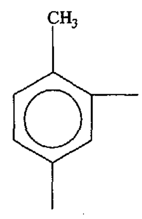 ;

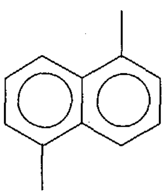 ; 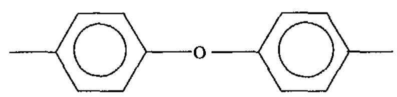 ; 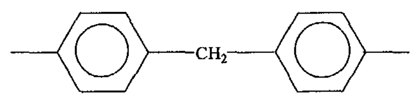 ;

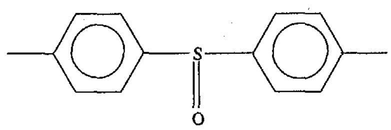 ; 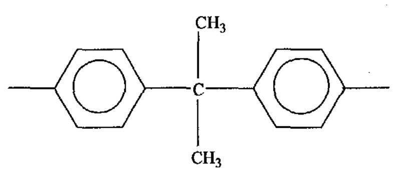 ;

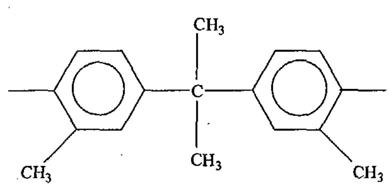 .

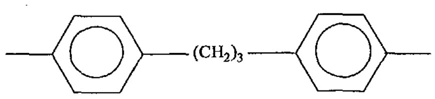 , 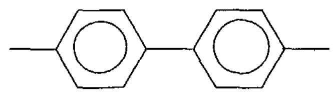

**14.** Composition selon l'une quelconque des revendications 11 à 13, **caractérisée par le fait que** $R_2$ est un groupe de formule :

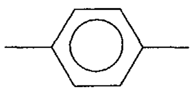 ; 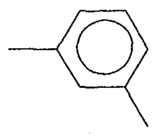 ; 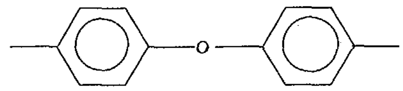

**15.** Composition selon l'une quelconque des revendications 11 à 14, **caractérisée par le fait que** $R_3$ est choisi parmi les groupes de formule :

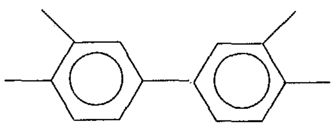 ; 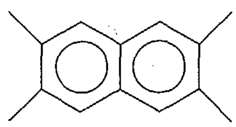 . 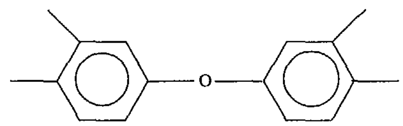 ;

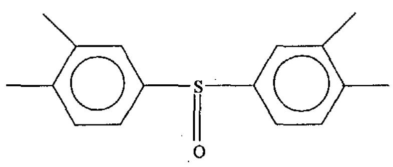 ; 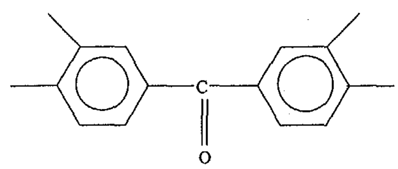 ;

**16.** Composition selon l'une quelconque des revendications 11 à 15, **caractérisée par le fait que** le polyimide-amide est obtenu par polymérisation du tolyulène diisocyanate et de l'anhydride triméllitique et comprend des motifs répétitifs de formule :

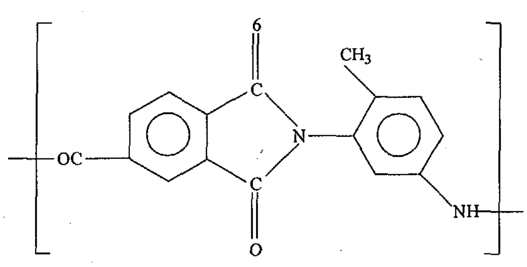

**17.** Composition selon l'une quelconque des revendications précédentes, **caractérisée par le fait que** les fibres rigides sensiblement rectilignes sont présentes en une teneur allant de 0,01 % à 10 % en poids, par rapport au poids total de la composition, de préférence allant de 0,1 % à 5 % en poids, et préférentiellement allant de 0,3 % à 3 % en poids.

**18.** Composition selon l'une quelconque des revendications précédentes, **caractérisée par le fait qu'**elle a une con-

sistance initiale $G^*_j$ allant de 1 x $10^2$ Pa à 1 x $10^5$ Pa, de préférence allant de 5 x $10^2$ Pa à 5 x $10^4$ Pa, et mieux allant de 6 x $10^2$ Pa à 9 x 103 Pa, mesurée sous une contrainte sinusoïdale à une fréquence de 1 Hz.

**19.** Composition selon l'une quelconque des revendications précédentes, **caractérisée par le fait qu'**elle a une élasticité initiale $\delta_i$ allant de 1 ° à 45 °, et mieux allant de 10° à 35°.

**20.** Composition selon l'une quelconque des revendications précédentes, **caractérisée par le fait qu'**elle a un seuil d'écoulement $\tau_c$ allant de 10 Pa à 3500 Pa, et de préférence allant de 20 Pa à 1000 Pa.

**21.** Composition selon l'une quelconque des revendications précédentes, **caractérisée par le fait qu'**elle comprend un agent épaississant thixotrope.

**22.** Composition selon l'une quelconque des revendications précédentes, **caractérisée par le fait que** la composition comprend un milieux aqueux.

**23.** Composition selon l'une quelconque des revendications précédentes, **caractérisée par le fait qu'**elle comprend de l'eau et éventuellement un solvant organique hydrophile.

**24.** Composition selon la revendication précédente, **caractérisée par le fait que** le solvant organique hydrophile est choisi parmi les monoalcools ayant de 2 à 5 atomes de carbone, les polyols ayant de 2 à 8 atomes de carbone, les cétones en $C_3$-$C_4$, les aldéhydes en $C_2$-$C_4$.

**25.** Composition selon la revendication 23 ou 24, **caractérisée par le fait que** l'eau ou le mélange d'eau et de solvant organique hydrophile est présent en une teneur allant de 0,1 % à 90 % en poids, par rapport au poids total de la composition, et de préférence de 0,1 % à 60 % en poids.

**26.** Composition selon l'une des revendications 21 à 25, **caractérisée par le fait que** l'agent épaississant thixotrope est un épaississant thixotrope de milieu aqueux choisi parmi les argiles hydrophiles, la gomme de carraghénane, la silice pyrogénée hydrophile.

**27.** Composition selon la revendication 26, **caractérisée par le fait que** l'argile hydrophile est choisie dans le groupe formé par les argiles de la famille des smectites, des vermiculites, de la stévensite, des chlorites.

**28.** Composition selon la revendication 26 ou 27, **caractérisée par le fait que** l'argile hydrophile est choisie dans le groupe formé par les montmorillonites, les hectorites, les bentonites, la beidellite, les saponites.

**29.** Composition selon l'une quelconque des revendications précédentes, **caractérisée par le fait qu'**elle comprend une phase grasse liquide.

**30.** Composition selon la revendication précédente, **caractérisée par le fait que** la phase grasse liquide comprend un corps gras choisi parmi les huiles, les solvants organiques, et leurs mélanges.

**31.** Composition selon la revendication 29 ou 30, **caractérisée par le fait qu'**elle comprend une huile choisie parmi les huiles d'origine minérale, animale, végétale ou synthétique, carbonées, hydrocarbonées, fluorées et/ou siliconées, seules ou en mélange.

**32.** Composition selon l'une quelconque des revendications 29 à 31, **caractérisée par le fait que** la phase grasse liquide représente de 0,1 % à 98 % en poids, par rapport au poids total de la composition, et de préférence de 1 à 80 % en poids.

**33.** Composition selon l'une quelconque des revendications 29à 32, **caractérisée par le fait qu'**elle comprend au moins une huile volatile ou un solvant organique volatile.

**34.** Composition selon la revendication précédente, **caractérisée par le fait que** l'huile volatile est choisie parmi l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, les alcanes ramifiés en $C_8$-$C_{16}$.

**35.** Composition selon la revendication 33 ou 34, **caractérisée par le fait que** l'huile volatile est présente en une teneur allant de 0,1 % à 98 % en poids, par rapport au poids total de la composition, de préférence de 1 % à 65 % en poids.

**36.** Composition selon l'une quelconque des revendications 29 à 35, **caractérisée par le fait qu'**elle comprend une huile non volatile.

**37.** Composition selon la revendication précédente, **caractérisée par le fait que** l'huile non volatile est présente en une teneur allant de 0,1 % à 80 % en poids, de préférence de 0,1 % à 50 % en poids, par rapport au poids total de la composition, et mieux de 0,1 % à 20 % en poids.

**38.** Composition selon l'une quelconque des revendications 21 et 29 à 37, **caractérisée par le fait que** l'agent épaississant thixotrope est un épaississant thixotrope de milieu huileux.

**39.** Composition selon la revendication précédente, **caractérisée par le fait que** l'agent épaississant thixotrope de milieu huileux est choisi parmi les argiles organophiles, les silices pyrogénées hydrophobes, les organopolysiloxanes élastomériques.

**40.** Composition selon l'une quelconque des revendications 21, 26 à 28, 38, 39, **caractérisée par le fait que** l'agent épaississant thixotrope est présent en une teneur allant de 0,5 % à 15 % en poids, par rapport au poids total de la composition, de préférence allant de 1 % à 15 % en poids, préférentiellement allant de 2 % à 10 % en poids, et plus préférentiellement allant de 2 % à 8 % en poids.

**41.** Composition selon l'une quelconque des revendications 21 à 40, **caractérisée par le fait qu'**elle comprend un agent épaississant additionnel.

**42.** Composition selon l'une quelconque des revendications 21 à 41, **caractérisée par le fait qu'**elle comprend un agent épaississant hydrophile additionnel.

**43.** Composition selon la revendication précédente, **caractérisée par le fait que** l'agent thixotrope additionnel est un polyuréthane associatif.

**44.** Composition selon l'une quelconque des revendications 21 à 43, **caractérisée par le fait qu'**elle comprend un agent épaississant lipophile additionnel.

**45.** Composition selon l'une quelconque des revendications 41 à 44, **caractérisée par le fait que** l'agent épaississant additionnel est présent en une teneur allant de 0,1 % à 5 % en poids, par rapport au poids total de la composition, et de préférence allant de 0,1 % à 3 % en poids.

**46.** Composition selon l'une quelconque des revendications précédentes, **caractérisée par le fait que** le polymère filmogène est choisi dans le groupe formé par les polymères vinyliques, les polymères acryliques, les polyuréthanes, les polyesters, les polyamides, les polyurées, les polymères cellulosiques.

**47.** Composition selon l'une quelconque des revendications précédentes, **caractérisée par le fait que** le polymère filmogène est présent sous forme de particules en dispersion aqueuse.

**48.** Composition selon l'une quelconque des revendications précédentes, **caractérisée par le fait que** le polymère filmogène est présent en une teneur en matières sèches de polymère allant de 0,1 % à 60 % en poids par rapport au poids total de la composition, de préférence de 0,5 % à 40 % en poids, et mieux de 1 % à 30 % en poids.

**49.** Composition selon l'une quelconque des revendications précédentes, **caractérisée par le fait que** la cire a une une température de fusion supérieure à 30°C et allant jusqu'à 120° C.

**50.** Composition selon l'une quelconque des revendications précédentes, **caractérisée par le fait que** la cire est choisie parmi la cire d'abeilles, la cire de lanoline, et les cires d'insectes de Chine; la cire de riz, la cire de Carnauba, la cire de Candellila, la cire d'Ouricury, la cire de fibres de liège, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides

gras et les glycérides concrets à 40°C, les cires obtenues par hydrogénation catalytique d'huiles animales ou végétales ayant des chaînes grasses, linéaires ou ramifiées, en C8-C32, les cires de silicone, les cires fluorées, et leur mélange.

**51.** Composition selon l'une quelconque des revendications précédentes, **caractérisée par le fait que** la cire a une dureté allant de 0,05 MPa à 15 MPa.

**52.** Composition selon l'une quelconque des revendications précédentes, **caractérisée par le fait que** la cire est présente en une teneur allant de 0,1 % à 50 % en poids, par rapport au poids total de la composition, de préférence de 0,5 % à 30 % en poids, et mieux de 1 % à 20 % en poids.

**53.** Composition selon l'une quelconque des revendications précédentes, **caractérisée par le fait qu'**elle comprend un corps gras pâteux.

**54.** Composition selon l'une quelconque des revendications précédentes, **caractérisée par le fait qu'**elle comprend un tensioactif.

**55.** Composition selon l'une quelconque des revendications précédentes, **caractérisée par le fait qu'**elle comprend des fibres courtes additionnelles, différentes des fibres rigides, et ayant une longueur inférieure à 0,8 mm , de préférence allant de 0,1 mm à 0,5 mm.

**56.** Composition selon l'une quelconque des revendications précédentes, **caractérisée par le fait qu'**elle comprend une matière colorante.

**57.** Composition selon la revendication précédente, **caractérisée par le fait que** la matière colorante est choisie parmi les pigments, les nacres, les colorants liposolubles, les colorants hydrosolubles.

**58.** Composition selon la revendication 53 ou 54, **caractérisée par le fait que** la matière colorante est présente en une teneur allant de 0,01 % à 30 % en poids, par rapport au poids total de la composition.

**59.** Composition selon l'une quelconque des revendications précédentes, **caractérisée par le fait qu'**elle comprend une charge.

**60.** Composition selon l'une quelconque des revendications précédentes, **caractérisée par le fait qu'**elle comprend un additif cosmétique choisi parmi les antioxydants, les conservateurs, les parfums, les neutralisants, les émollients, les hydratants, les vitamines, les filtres solaires, les agents plastifiants, les agents de coalescence.

**61.** Composition selon l'une quelconque des revendications précédentes, **caractérisée par le fait qu'**elle est sous la forme de composition de revêtement des cils, de produit pour sourcils, d'eye-liner, de produit pour les lèvres, de fard à joues ou à paupières, de fond de teint, de produit de maquillage du corps, de produit anti-cernes, de vernis à ongles, de produit de soin de la peau.

**62.** Composition selon l'une quelconque des revendications précédentes, **caractérisée par le fait qu'**elle est une composition de soin ou de maquillage des fibres kératiniques.

**63.** Composition selon l'une quelconque des revendications précédentes, **caractérisée par le fait qu'**elle est un mascara.

**64.** Procédé cosmétique de maquillage et de soin des matières kératiniques comprenant l'application sur les matières kératiniques d'une composition selon l'une quelconque des revendications précédentes.

**65.** Procédé cosmétique de maquillage et de soin des cils comprenant l'application sur les cils d'une composition selon l'une quelconque des revendications 1 à 60 et 62 ou 63.

**66.** Utilisation de fibres rigides, sensiblement rectilignes, dans une composition comprenant, dans un milieu physiologiquement acceptable, au moins un polymère filmogène et/ou au moins une cire, la composition ayant un comportement plastique thixotrope, pour obtenir un dépôt, notamment un maquillage, homogène sur les matières kératiniques.

**67.** Utilisation d'un mascara comprenant une composition selon l'une quelconque des revendications 1 à 60, pour obtenir un allongement dans le prolongement des cils et/ou pour mimer le prolongement des cils.

**68.** Utilisation de fibres rigides, sensiblement rectilignes, dans une composition comprenant, dans un milieu physiologiquement acceptable, au moins un polymère filmogène et/ou au moins une cire, la composition ayant un comportement plastique thixotrope, pour obtenir un allongement dans le prolongement des cils et/ou pour mimer le prolongement des cils.

Office européen des brevets

# RAPPORT DE RECHERCHE EUROPEENNE

Numéro de la demande
EP 03 29 2137

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | FR 2 675 995 A (OREAL)<br>6 novembre 1992 (1992-11-06)<br>* exemples 1-3 *<br>* revendications *<br>* page 4, ligne 1-5 *<br>* page 1, ligne 28-37 *<br>* page 2, ligne 12-41 *<br>* page 2, ligne 28 *<br>--- | 1-61,64,<br>66 | A61K7/02<br>A61K7/021<br>A61K7/025<br>A61K7/031<br>A61K7/032<br>A61K7/043<br>A61K7/48<br>A61K7/06 |
| X | EP 0 714 653 A (OREAL)<br>5 juin 1996 (1996-06-05)<br>* revendications 1,3,4 *<br>* page 2, ligne 16-25 *<br>* page 3, ligne 2-9 *<br>--- | 1-61,64,<br>66 | |
| X | US 6 156 325 A (FARER ALAN ET AL)<br>5 décembre 2000 (2000-12-05)<br>* revendication 1 *<br>* colonne 2, ligne 64 - colonne 4, ligne 33 *<br>* colonne 5, ligne 48,49 *<br>--- | 1-61,64,<br>66 | |
| | | | **DOMAINES TECHNIQUES RECHERCHES (Int.Cl.7)** |
| X | WO 02 41851 A (BLIN XAVIER ;OREAL (FR); JAGER LEZER NATHALIE (FR))<br>30 mai 2002 (2002-05-30)<br>* exemples 1-3 *<br>* page 23, ligne 18-29 *<br>--- | 1-61,64,<br>66 | A61K |
| X | FR 2 816 831 A (OREAL)<br>24 mai 2002 (2002-05-24)<br>* exemples 1,2 *<br>* page 19, ligne 41 - page 20, ligne 8 *<br>--- | 1-61,64,<br>66 | |
| X | FR 2 816 832 A (OREAL)<br>24 mai 2002 (2002-05-24)<br>* exemples 3,4 *<br>---<br>-/-- | 1-68 | |

Le présent rapport a été établi pour toutes les revendications

| Lieu de la recherche | Date d'achèvement de la recherche | Examinateur |
|---|---|---|
| MUNICH | 21 janvier 2004 | Hauss, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..................................................................
& : membre de la même famille, document correspondant

EPO FORM 1503 03.82 (P04C02)

Office européen des brevets

# RAPPORT DE RECHERCHE EUROPEENNE

Numéro de la demande
EP 03 29 2137

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | FR 1 529 329 A (MAX FACTOR & CO) 14 juin 1968 (1968-06-14) * revendications * * exemples 1,2 * * page 2 * | 1-61,64, 66 | |
| X | WO 92 05762 A (BOOTS CO PLC) 16 avril 1992 (1992-04-16) * exemples 1-12,14-17 * * revendications * * page 1, ligne 5-23 * * page 5, ligne 1-14 * | 1-61,64, 66 | |
| X | EP 1 064 930 A (OREAL) 3 janvier 2001 (2001-01-03) * exemples 1,2 * * revendications 1,10,19,21,22,27 * * alinéas [0021]-[0029] * * alinéas [0032]-[0045] * * alinéas [0061],[0062] * | 1-61,64, 66 | |
| X | EP 1 157 683 A (OREAL) 28 novembre 2001 (2001-11-28) * revendications 1,7 * * exemples 1-3 * | 1-61,64, 66 | DOMAINES TECHNIQUES RECHERCHES (Int.Cl.7) |
| X | EP 1 201 221 A (OREAL) 2 mai 2002 (2002-05-02) * exemple 3 * * revendications * | 1-68 | |
| X | EP 1 066 814 A (OREAL) 10 janvier 2001 (2001-01-10) * revendications 1,8,13 * * exemple 3 * | 1-68 | |
| | -/-- | | |

Le présent rapport a été établi pour toutes les revendications

| Lieu de la recherche | Date d'achèvement de la recherche | Examinateur |
|---|---|---|
| MUNICH | 21 janvier 2004 | Hauss, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 03 29 2137

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | DATABASE CA [en ligne] CHEMICAL ABSTRACTS SERVICE, COLUMBUS, OHIO, US; KOSUGI, MASAKI ET AL: "Mascaras containing film-forming resins, oils, and fibers" retrieved from STN Database accession no. 136:406591 XP002243885 * abrégé * -& JP 2002 154932 A (KOSEI CO., LTD., JAPAN) 28 mai 2002 (2002-05-28) --- | 1-68 | |
| X,D | PATENT ABSTRACTS OF JAPAN vol. 1998, no. 02, 30 janvier 1998 (1998-01-30) -& JP 09 263518 A (KOSE CORP), 7 octobre 1997 (1997-10-07) * abrégé * --- | 1-68 | |
| X | US 2002/102226 A1 (PARDO JANET ET AL) 1 août 2002 (2002-08-01) * revendications 1,15-21 * * exemples * * alinéas [0010],[0017]-[0020],[0023],[0024] * --- | 1-68 | DOMAINES TECHNIQUES RECHERCHES (Int.Cl.7) |
| | -/-- | | |

Le présent rapport a été établi pour toutes les revendications

| Lieu de la recherche | Date d'achèvement de la recherche | Examinateur |
|---|---|---|
| MUNICH | 21 janvier 2004 | Hauss, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 03 29 2137

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | DATABASE CA [en ligne]<br>CHEMICAL ABSTRACTS SERVICE, COLUMBUS, OHIO, US;<br>HASEGAWA, MAKOTO: "Eye makeups and hair preparations containing iridescent oblong polymer powder"<br>retrieved from STN<br>Database accession no. 133:366204<br>XP002267594<br>* abrégé *<br>-& JP 2000 319131 A (NIPPON SHIKIZAI KOGYO KENKYUSHO K. K., JAPAN)<br>21 novembre 2000 (2000-11-21)<br>----- | 1-68 | |

DOMAINES TECHNIQUES RECHERCHES (Int.Cl.7)

Le présent rapport a été établi pour toutes les revendications

| Lieu de la recherche | Date d'achèvement de la recherche | Examinateur |
|---|---|---|
| MUNICH | 21 janvier 2004 | Hauss, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 03 29 2137

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

21-01-2004

| Document brevet cité au rapport de recherche | | Date de publication | Membre(s) de la famille de brevet(s) | | Date de publication |
|---|---|---|---|---|---|
| FR 2675995 | A | 06-11-1992 | FR | 2675995 A1 | 06-11-1992 |
| | | | CA | 2109423 A1 | 03-11-1992 |
| | | | DE | 69203602 D1 | 24-08-1995 |
| | | | DE | 69203602 T2 | 04-04-1996 |
| | | | EP | 0583347 A1 | 23-02-1994 |
| | | | ES | 2077418 T3 | 16-11-1995 |
| | | | WO | 9219282 A1 | 12-11-1992 |
| | | | JP | 6507166 T | 11-08-1994 |
| | | | US | 5370866 A | 06-12-1994 |
| EP 0714653 | A | 05-06-1996 | FR | 2727625 A1 | 07-06-1996 |
| | | | CA | 2164248 A1 | 03-06-1996 |
| | | | DE | 69500369 D1 | 24-07-1997 |
| | | | DE | 69500369 T2 | 02-10-1997 |
| | | | EP | 0714653 A1 | 05-06-1996 |
| | | | ES | 2105855 T3 | 16-10-1997 |
| | | | JP | 2726025 B2 | 11-03-1998 |
| | | | JP | 8208438 A | 13-08-1996 |
| | | | US | 5725866 A | 10-03-1998 |
| US 6156325 | A | 05-12-2000 | US | 2001002253 A1 | 31-05-2001 |
| WO 0241851 | A | 30-05-2002 | FR | 2816830 A1 | 24-05-2002 |
| | | | WO | 0241851 A1 | 30-05-2002 |
| FR 2816831 | A | 24-05-2002 | FR | 2816831 A1 | 24-05-2002 |
| | | | EP | 1339375 A1 | 03-09-2003 |
| | | | WO | 0241852 A1 | 30-05-2002 |
| FR 2816832 | A | 24-05-2002 | FR | 2816832 A1 | 24-05-2002 |
| | | | EP | 1339376 A1 | 03-09-2003 |
| | | | WO | 0241855 A1 | 30-05-2002 |
| FR 1529329 | A | 14-06-1968 | AUCUN | | |
| WO 9205762 | A | 16-04-1992 | AT | 125442 T | 15-08-1995 |
| | | | AU | 8520191 A | 28-04-1992 |
| | | | DE | 69111621 D1 | 31-08-1995 |
| | | | DE | 69111621 T2 | 21-12-1995 |
| | | | WO | 9205762 A1 | 16-04-1992 |
| | | | EP | 0621771 A1 | 02-11-1994 |
| | | | ES | 2074726 T3 | 16-09-1995 |
| | | | US | 5330750 A | 19-07-1994 |
| EP 1064930 | A | 03-01-2001 | FR | 2795640 A1 | 05-01-2001 |
| | | | AT | 212828 T | 15-02-2002 |

EPO FORM P0460

Pour tout renseignement concernant cette annexe : voir Journal Officiel de l'Office européen des brevets, No.12/82

## ANNEXE AU RAPPORT DE RECHERCHE EUROPEENNE RELATIF A LA DEMANDE DE BREVET EUROPEEN NO.

EP 03 29 2137

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

21-01-2004

| Document brevet cité au rapport de recherche | Date de publication | Membre(s) de la famille de brevet(s) | Date de publication |
|---|---|---|---|
| EP 1064930 A | | BR 0002459 A | 13-03-2001 |
| | | DE 60000072 D1 | 21-03-2002 |
| | | DE 60000072 T2 | 18-07-2002 |
| | | EP 1064930 A1 | 03-01-2001 |
| | | ES 2172498 T3 | 01-10-2002 |
| | | JP 2001048736 A | 20-02-2001 |
| | | US 2003031642 A1 | 13-02-2003 |
| EP 1157683 A | 28-11-2001 | FR 2809009 A1 | 23-11-2001 |
| | | EP 1157683 A2 | 28-11-2001 |
| | | JP 2001335462 A | 04-12-2001 |
| | | US 2002031533 A1 | 14-03-2002 |
| EP 1201221 A | 02-05-2002 | FR 2815847 A1 | 03-05-2002 |
| | | BR 0105682 A | 25-06-2002 |
| | | CN 1350838 A | 29-05-2002 |
| | | EP 1201221 A2 | 02-05-2002 |
| | | JP 2002145739 A | 22-05-2002 |
| | | US 2002098217 A1 | 25-07-2002 |
| EP 1066814 A | 10-01-2001 | FR 2795950 A1 | 12-01-2001 |
| | | EP 1066814 A1 | 10-01-2001 |
| | | WO 0103653 A1 | 18-01-2001 |
| | | JP 2001048750 A | 20-02-2001 |
| | | US 6491931 B1 | 10-12-2002 |
| JP 2002154932 A | 28-05-2002 | AUCUN | |
| JP 09263518 A | 07-10-1997 | AUCUN | |
| US 2002102226 A1 | 01-08-2002 | AU 5456100 A | 18-12-2000 |
| | | WO 0072809 A1 | 07-12-2000 |
| JP 2000319131 A | 21-11-2000 | AUCUN | |

EPO FORM P0460

Pour tout renseignement concernant cette annexe : voir Journal Officiel de l'Office européen des brevets, No.12/82